(19) Europäisches Patentamt
European Patent Office
Office européen des brevets

(11) EP 4 653 089 A1

(12) EUROPEAN PATENT APPLICATION
published in accordance with Art. 153(4) EPC

(43) Date of publication:
26.11.2025 Bulletin 2025/48

(21) Application number: 23917777.7

(22) Date of filing: 27.12.2023

(51) International Patent Classification (IPC):
*B01J 27/24* (2006.01) *B01J 37/04* (2006.01)
*B01J 37/08* (2006.01) *B01J 37/16* (2006.01)
*C01B 32/40* (2017.01) *C07B 61/00* (2006.01)
*C07C 29/157* (2006.01) *C07C 31/04* (2006.01)

(52) Cooperative Patent Classification (CPC):
B01J 27/24; B01J 37/04; B01J 37/08; B01J 37/16;
C01B 32/40; C07B 61/00; C07C 29/157;
C07C 31/04

(86) International application number:
PCT/JP2023/046981

(87) International publication number:
WO 2024/154567 (25.07.2024 Gazette 2024/30)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 20.01.2023 JP 2023007378

(71) Applicant: Japan Science and Technology Agency
Kawaguchi-shi, Saitama 332-0012 (JP)

(72) Inventors:
- HOSONO, Hideo
  Tokyo 152-8550 (JP)
- KITANO, Masaaki
  Tokyo 152-8550 (JP)
- SUGIYAMA, Hironobu
  Tokyo 152-8550 (JP)
- YOKOYAMA, Toshiharu
  Tokyo 152-8550 (JP)
- XING, Feilong
  Tokyo 152-8550 (JP)

(74) Representative: Gilani, Anwar
Venner Shipley LLP
406 Science Park
Milton Road
Cambridge CB4 0WW (GB)

(54) INTERMETALLIC COMPOUND, CATALYST, PRODUCTION METHOD FOR METHANOL, PRODUCTION METHOD FOR CARBON MONOXIDE, AND PRODUCTION METHOD FOR INTERMETALLIC COMPOUND

(57) Provided is an intermetallic compound that is excellent in terms of catalytic performance. An intermetallic compound according to the present embodiment includes a crystal lattice in which a first metal atom and the second metal atom are adjacent to each other. The first metal atom is at least one selected from the group consisting of Pd, Rh, and Ir. The second metal atom is Mo. An X-ray diffraction spectrum before temperature-programmed desorption measurement has a first peak between 42° and 44°, and a second peak between 55° and 58° in terms of a diffraction angle 20. After the temperature-programmed desorption measurement, the intensity of the first peak and the intensity of the second peak are equal to or lower than those before the temperature-programmed desorption measurement, and the BET specific surface area thereof is at least 1 $m^2/g$.

EP 4 653 089 A1

FIG. 6

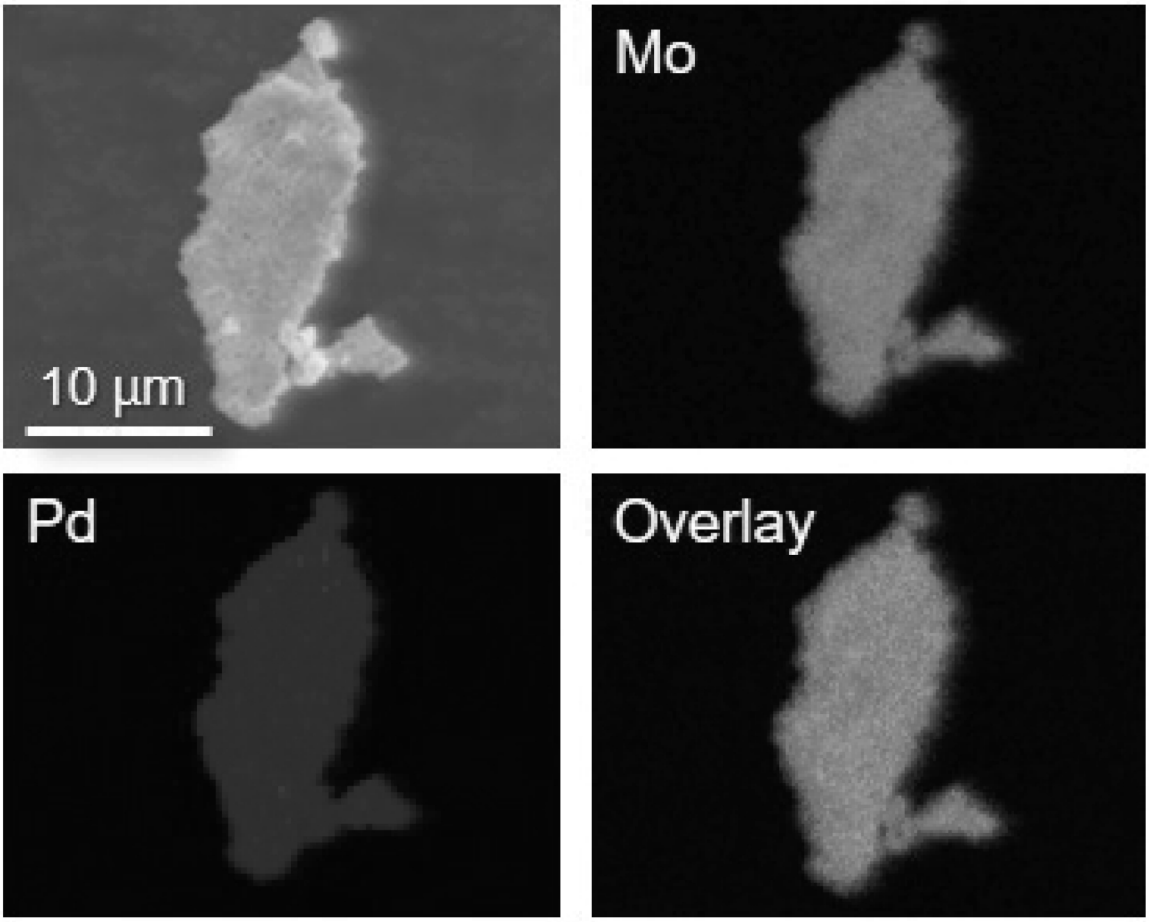

## Description

TECHNICAL FIELD

The present invention relates to an intermetallic compound, a catalyst, a method for producing methanol, a method for producing carbon monoxide, and a method for producing an intermetallic compound.

Priority is claimed on Japanese Patent Application No. 2023-007378, filed January 20, 2023, the content of which is incorporated herein by reference.

BACKGROUND ART

Processes for synthesizing methanol, which can be used as an energy carrier and the like, from $CO_2$, a greenhouse gas, are under development worldwide. Conventionally, research has been conducted on synthesizing methanol by hydrogenating $CO_2$ under high temperature and high pressure using Cu catalysts, Pd catalysts, etc.

However, the hydrogenation reaction from $CO_2$ to methanol is an exothermic reaction, and it is thermodynamically unfavorable to carry out the reaction at high temperatures. Therefore, development of catalysts that enable methanol synthesis at low temperatures is underway.

Non-Patent Document 1 discloses $MoS_2$ nanosheets that enable methanol synthesis from $CO_2$ at low temperatures.

Citation List

Non-Patent Document

Non-Patent Document 1: J. Hu, L. Yu, J. Deng, Y. Wang, K. Cheng, C. Ma, Q. Zhang, W. Wen, S. Yu, Y. Pan, J. Yang, H. Ma, F. Qi, Y. Wang, Y. Zheng, M. Chen, R. Huang, S. Zhang, Z. Zhao, J. Mao, X. Meng, Q. Ji, G. Hou, X. Han, X. Bao, Y. Wang, D. Deng, Nat Catal 2021, 4, 242-250.

SUMMARY OF INVENTION

Technical Problem

However, the catalyst of Non-Patent Document 1 has not always been fully satisfactory.

Solution to Problem

Under these circumstances, the inventors of the present application have made extensive efforts to discover an intermetallic compound capable of synthesizing methanol from $CO_2$ or CO at low temperatures, and furthermore having high conversion efficiency and stable reusability, which led to the development of the present invention. That is, the present invention provides an intermetallic compound that is excellent in catalytic performance, a catalyst that utilizes the intermetallic compound, a method for producing methanol, a method for producing carbon monoxide, and a method for producing an intermetallic compound.

More specifically, the present invention proposes the following means.

<1> An intermetallic compound of a first aspect of the present invention, comprising a first metal atom and a second metal atom and having a crystal lattice in which the first metal atom and the second metal atom are adjacent to each other,

wherein the first metal atom is at least one selected from the group consisting of Pd, Rh, and Ir,
the second metal atom is Mo,
the X-ray diffraction spectrum before the temperature-programmed desorption measurement has a first peak between 42° and 44° and a second peak between 55° and 58° in terms of a diffraction angle 2θ,
a position of the first peak and a position of the second peak are different from a position of a peak of the elemental form of the first metal atom and a position of a peak of the elemental form of the second metal atom,
after the temperature-programmed desorption measurement, the intensity of the first peak and the intensity of the second peak are equal to or lower than those before the temperature-programmed desorption measurement, and
the intermetallic compound has a BET specific surface area of 1 $m^2$/g or more.

<2> A second aspect of the present invention is the intermetallic compound of the first aspect, wherein at least a portion of the crystal lattice has a hexagonal close-packed structure.
<3> A third aspect of the present invention is the intermetallic compound of the first aspect or the second aspect, wherein a volume fraction of the hexagonal close-packed structure with respect to a total volume of the intermetallic compound is 50% or more.
<4> A fourth aspect of the present invention is the intermetallic compound of any one of the first to third aspects, wherein a molar fraction of the first metal atoms with respect to total moles of the first metal atoms and the second metal atoms contained in the intermetallic compound is 5 mol% or more and 95 mol% or less.
<5> A fifth aspect of the present invention is the intermetallic compound of any one of the first to third aspects, wherein a molar fraction of the first metal atoms with respect to total moles of the first metal atoms and the second metal atoms contained in the intermetallic compound is 10 mol% or more and 90 mol% or less.
<6> A sixth aspect of the present invention is the intermetallic compound of any one of the first to fifth aspects, wherein a molar ratio of the first metal atoms to the second metal atoms is from 30:70 to 70:30.
<7> A seventh aspect of the present invention is the intermetallic compound of the sixth aspect, wherein a molar ratio of the first metal atoms to the second metal atoms is from 40:60 to 65:35.
<8> An eighth aspect of the present invention is the intermetallic compound of the seventh aspect, wherein a molar ratio of the first metal atoms to the second metal atoms is from 50:50 to 60:40.
<9> A ninth aspect of the present invention is the intermetallic compound of any one of the first to eighth aspects, comprising a multi-coordinating atom that exhibits multi-coordination.
<10> A tenth aspect of the present invention is the intermetallic compound of the ninth aspect, wherein the multi-coordinating atom is at least one selected from the group consisting of nitrogen (N), oxygen (O), carbon (C), and boron (B).
<11> An eleventh aspect of the present invention is the intermetallic compound of any one of the first to tenth aspects, comprising at least one selected from the group consisting of an oxide of at least one of the first metal atom and the second metal atom, and a nitride of at least one of the first metal atom and the second metal atom.
<12> A catalyst of a twelve aspect comprising:

the intermetallic compound of any one of the first to the eleventh aspects, and
a support that carries the intermetallic compound.

<13> A method for producing methanol of a 13th aspect of the present invention, comprising bringing hydrogen and at least one of carbon monoxide and carbon dioxide into contact with the intermetallic compound of any one of the first to eleventh aspects
<14> A method for producing carbon monoxide of a 14th aspect of the present invention, comprising bring hydrogen and carbon dioxide into contact with the intermetallic compound of any one of the first to eleventh aspects.
<15> A method for producing an intermetallic compound of a 15th aspect of the present invention,

wherein the intermetallic compound includes a first metal atom and a second metal atom,
the first metal atom is at least one selected from the group consisting of Pd, Rh, and Ir, and
the second metal atom is Mo,
the method for producing an intermetallic compound including:

a first step of mixing a first compound containing the first metal atom and a second compound containing the second metal atom, dehydrating the mixture to obtain a dehydrated-product, and then carbonizing the dehydrated-product to obtain a carbide;
a second step of calcining the carbide obtained in the first step under air to obtain a calcined-product; and
a third step of subjecting the calcined-product obtained in the second step to a nitridation treatment in an $NH_3$ atmosphere.

<16> A method for producing an intermetallic compound of a 16th aspect of the present invention is the method for producing an intermetallic compound of the 15th aspect,
wherein in the first step, the first compound and the second compound may be mixed in the presence of citric acid.
<17> A method for producing an intermetallic compound of an 17th aspect of the present invention is the method for producing an intermetallic compound of the 15th aspect,
wherein in the first step, the first compound and the second compound may be mixed in the presence of polyvinylpyrrolidone.

Advantageous Effects of Invention

According to the above-mentioned aspects of the present invention, it is possible to provide an intermetallic compound having excellent catalytic performance, a catalyst utilizing the same, a method for producing methanol, a method for producing carbon monoxide, and a method for producing an intermetallic compound.

BRIEF DESCRIPTION OF DRAWINGS

FIG. 1 is a flow chart of a method for producing an intermetallic compound according to the present embodiment.
FIG. 2 shows X-ray diffraction spectra of Examples 1 to 4, Comparative Example 2, and $Mo_2N$ (ICSD No.251625) in ICSD.
FIG. 3 shows X-ray diffraction spectra of hexagonal close-packed PdMo (hcp-PdMo) of Example 2 and the same in ICSD.
FIG. 4 shows X-ray diffraction spectra of Example 2 before and after temperature-programmed desorption measurement, and Mo (No. 52267) and Pd (No. 52251) in ICSD.
FIG. 5 shows X-ray diffraction spectra of the catalyst of Example 2 immediately after preparation and the catalyst of Example 2 stored in air for 10 days after preparation.
FIG. 6 is an elemental mapping image of the catalyst of Example 2.
FIG. 7 is a graph showing the results of temperature-programmed desorption measurement of the catalyst of Example 2.
FIG. 8 is a graph showing the relationship between the Pd content in the catalyst and the methanol synthesis rate when carbon dioxide is used.
FIG. 9 is a graph showing the relationship between the reaction temperature and the methanol synthesis rate in the catalysts of Example 1 and Example 2.
FIG. 10 is a graph showing the relationship between the reaction temperature and the methanol synthesis rate when the pressure conditions are changed.
FIG. 11 is a graph showing the relationship between the reaction time and the methanol synthesis rate in the catalyst of Example 1.
FIG. 12 shows X-ray diffraction spectra of the catalyst according to the present embodiment before and after the continuous reaction.
FIG. 13 is a graph showing the relationship between the reaction temperature and the carbon monoxide synthesis rate in the catalysts of Example 1 and Comparative Example 1.
FIG. 14 is a graph showing the relationship between the reaction temperature and the methanol synthesis rate by $CO_2$ hydrogenation in the catalysts of Example 1 and Examples 7 to 9.
FIG. 15 is a graph showing the relationship between the selectivity to methanol and the reaction temperature in the catalyst of Example 1.
FIG. 16 is a graph showing the relationship between the selectivity to methanol and the reaction temperature for the catalyst of Example 8.

DESCRIPTION OF EMBODIMENTS

Hereinafter, an intermetallic compound, a catalyst, a method for producing methanol, a method for producing carbon monoxide, and a method for producing an intermetallic compound according to the embodiments will be described.

<Intermetallic Compound>

The intermetallic compound of the present embodiment is an intermetallic compound containing a first metal atom and a second metal atom, and includes a crystal lattice in which the first metal atom and the second metal atom are adjacent to each other, wherein the first metal atom is one or more selected from the group consisting of Pd, Rh, and Ir, the second metal atom is Mo, and the X-ray diffraction spectrum before the temperature-programmed desorption measurement has a first peak between 42° and 44° and a second peak between 55° and 58° in terms of a diffraction angle $2\theta$, and the positions of the first peak and the second peak are different from the positions of the peaks of the elemental form of the first metal atom and the peaks of the elemental form of the second metal atom, and after the temperature-programmed desorption measurement, the intensity of the first peak and the intensity of the second peak are equal to or lower than those before the temperature-programmed desorption measurement, and the BET specific surface area is 1 $m^2/g$ or more. Here, the intermetallic compound is a compound composed of two or more metals. Hereinafter, the intermetallic compound of the present embodiment will be described.

(Chemical Composition)

The intermetallic compound of the present embodiment contains a first metal atom and a second metal atom, which will be described later. The chemical composition of the intermetallic compound of the present embodiment will be described below.

"First Metal Atom"

In the intermetallic compound of the present embodiment, the first metal atom is one or more selected from the group consisting of Pd, Rh, and Ir. By employing one or more selected from the group consisting of Pd, Rh, and Ir as the first metal atom, the intermetallic compound of the present embodiment can exhibit high conversion efficiency.

"Second Metal Atom"

In the intermetallic compound of the present embodiment, the second metal atom is Mo. When the first metal atom is one or more selected from the group consisting of Pd, Rh, and Ir, and the second metal atom is Mo, the intermetallic compound of the present embodiment can exhibit high conversion efficiency in the synthesis of methanol from $CO_2$ or CO.

"Molar Fraction of First Metal Atom With Respect to Total Moles of First Metal Atom and Second Metal Atom"

A molar percentage (molar fraction) of the first metal atom with respect to the total moles of the first metal atom and the second metal atom contained in the intermetallic compound of the present embodiment is preferably 5 mol% or more and 95 mol% or less. In addition, when there are two or more kinds of first metal atoms, the moles of the first metal atom is the sum of the moles of those atoms. More preferably, the molar fraction of the first metal atom is 10 mol% or more and 90 mol% or less. Further preferably, the molar fraction of the first metal atom is 10 mol% or more and 60 mol% or less. When the mole fraction exceeds 60 mol%, the proportion of crystal lattices where the first metal atom and the second metal atom are adjacent to each other decreases, and as a result, the configuration does not necessarily remain preferable.

When the molar fraction of the first metal atoms to the total moles of the first metal atoms and the second metal atoms contained in the intermetallic compound is 5 mol% or more and 95 mol% or less, the proportion of crystal lattices where the first metal atoms and the second metal atoms are adjacent to each other increases, and the conversion efficiency of the intermetallic compound in the synthesis of methanol from $CO_2$ or CO can be improved.

In addition, when the intermetallic compound of the present embodiment contains other atoms (e.g., one or more types of multi-coordinating atoms) other than both the first metal atom and the second metal atom, the molar fraction of the other atoms to the total moles of the first metal atom and the second metal atom (i.e., 100 mol%) is preferably 30.0 mol% or less, more preferably 20.0 mol% or less.

"Molar Ratio of First Metal Atom to Second Metal Atom"

The molar ratio of the first metal atom to the second metal atom in the intermetallic compound of the present embodiment is preferably 30:70 to 70:30. A more preferable molar ratio of the first metal atom to the second metal atom is 40:60 to 65:35. A further preferable molar ratio of the first metal atom to the second metal atom is 50:50 to 60:40. If the molar ratio of the first metal atom to the second metal atom in the intermetallic compound of the present embodiment is 30:70 to 70:30, the number of crystal lattices in which the first metal atom and the second metal atom are adjacent to each other increases, and the conversion efficiency of the intermetallic compound can be further improved in the synthesis of methanol from $CO_2$ or CO.

"Multi-Coordinating Atom that Exhibits Multi-Coordination"

The intermetallic compound of the present embodiment preferably contains a multi-coordinating atom that exhibits multi-coordination. When the intermetallic compound of the present embodiment contains a multi-coordinating atom that exhibits multi-coordination, the stability of the intermetallic compound in terms of the crystal structure is improved. Here, the multi-coordinating atom refers to an atom having two or more nearest neighbor atoms in the crystal. The multi-coordinating atom is preferably one or more selected from the group consisting of N, O, C, and B. The multi-coordinating atom is preferably N and O. Since the stability of the intermetallic compound of the present embodiment in terms of the crystal structure is further improved, N is particularly preferred as the multi-coordinating atom.

The total content of the multi-coordinating atoms with respect to the total mass of the intermetallic compound of the present embodiment is preferably 30.0 mol% or less. More preferably, the total content of the multi-coordinating atoms is 20.0 mol% or less. Since the multi-coordinating atoms may not be contained, the lower limit of the total content of the multi-coordinating atoms is 0 mass%. More preferably, the total content of the multi-coordinating atoms is 0.1 mol% or more. When the multi-coordinating atom is N, the N content in the intermetallic compound is preferably 3 mol% to 15 mol%. When the N content in the intermetallic compound of the present embodiment is 3 mol% to 15 mol%, the stability of the intermetallic compound is further improved.

When an intermetallic compound is composed of one or more first metal atoms, a second metal atom, and one or more multi-coordinating atoms, the intermetallic compound can be represented as $A_xB_yC_z$, where A is the first metal atom, B is the second metal atom, C is the multi-coordinating atom, $0.05 \leq x \leq 0.95$, $0.05 \leq y \leq 0.95$, $x + y = 1.00$, and $0 \leq z \leq 0.3$.

The molar fraction of the first metal atoms to the total moles of the first metal atoms and the second metal atoms contained in the intermetallic compound and the molar ratio of the first metal atoms to the second metal atoms in the intermetallic compound can be evaluated by analyzing the surface of a sample with an electron probe microanalyzer (EPMA) and determining the content ratio of the detected atoms by the ZAF method. Similarly, the presence or absence of a multi-coordinating atom and the content of the multi-coordinating atom can be evaluated by analyzing the surface of a sample with an EPMA and determining the content ratio of the detected atoms by the ZAF method.

(X-ray Diffraction Spectrum)

The X-ray diffraction spectrum of the intermetallic compound of the present embodiment before the temperature-programmed desorption measurement has a first peak between 42° and 44° and a second peak between 55° and 58° in terms of a diffraction angle 2$\theta$. The presence of the first peak between 42° and 44° and the second peak between 55° and 58° allows the first metal atoms and the second metal atoms to be arranged in a manner that promotes the hydrogenation reaction of $CO_2$ or CO, thereby improving the conversion efficiency in the synthesis of methanol from $CO_2$ or CO.

In the X-ray diffraction spectrum of the intermetallic compound of the present embodiment before the temperature-programmed desorption measurement, the positions of the first peak and the second peak are different from the positions of the peaks of the elemental form of the first metal atom and the elemental form of the second metal atom. The distinct peak positions indicate that the intermetallic compound has a unique crystal structure and bonding pattern, differing from the elemental forms of its constituent metals, enabling unique catalytic properties. An intermetallic compound is a compound having a crystal structure in which the two constituent elements are bonded in a simple integer ratio and are regularly arranged.

As the peaks of the elemental form of the first metal atom and the elemental form of the second metal atom, data in the Inorganic Crystal Structure Database (ICSD) can be used.

In the X-ray diffraction spectrum of the intermetallic compound of the present embodiment after the temperature-programmed desorption measurement, the intensity of the first peak and the intensity of the second peak are equal to or decreased compared to those before the temperature-programmed desorption measurement. That is, the intensity of the first peak is equal to or decreased by performing the temperature-programmed desorption measurement. Similarly, the intensity of the second peak is equal to or decreased by performing the temperature-programmed desorption measurement.

When the X-ray diffraction spectrum of the intermetallic compound of the present embodiment before the temperature-programmed desorption measurement has a first peak between 42° and 44° and a second peak between 55° and 58° in terms of a diffraction angle 2$\theta$, and in the X-ray diffraction spectrum of the intermetallic compound of the present embodiment after the temperature-programmed desorption measurement, the intensities of the first peak and the second peak are equal to or decreased (especially decreased) compared to those before the temperature-programmed desorption measurement, the intermetallic compound of the present embodiment, which exhibits such physical properties, has a crystal structure that is likely to promote the reaction in the hydrogenation reaction of $CO_2$ or CO. This can improve the conversion efficiency of the intermetallic compound in the synthesis of methanol from $CO_2$ or CO.

The X-ray diffraction spectrum of the intermetallic compound of the present embodiment before the temperature-programmed desorption measurement can be obtained by measuring a sample using, for example, an X-ray diffractometer (for example, an X-ray diffractometer MiniFlex 600 manufactured by RIGAKU Corporation) with an X-ray source of CuK$\alpha$, a measurement angle range of 20° to 70°, a measurement temperature of room temperature (25°C), a scan speed of 0.06 seconds/step, and a scan step of 0.01°. The X-ray diffraction spectrum of the intermetallic compound of the present embodiment after the temperature-programmed desorption measurement can be obtained by measuring the sample after the temperature-programmed desorption measurement, which will be described later, under the same conditions as the X-ray diffraction measurement conditions of the intermetallic compound of the present embodiment before the temperature-programmed desorption measurement.

When the intermetallic compound of the present embodiment is heated in an Ar atmosphere from room temperature (25°C) to 1000° at a rate of 10°C/min and then held at 1000°C for 1 hour, the gas species desorbed during this process can be confirmed by analyzing the gas species using a mass spectrometer attached to a catalyst analyzer (e.g., BELCAT II manufactured by MicrotracBEL Corporation). An example of the intermetallic compound after temperature-programmed desorption measurement is an intermetallic compound after heating in an Ar atmosphere from room temperature (25°C) to 1000°C at a rate of 10°C/min and then held at 1000°C for 1 hour.

(Crystal Lattice in Which First Metal Atom and Second Metal Atom Are Adjacent to Each Other)

The intermetallic compound of the present embodiment includes a crystal lattice in which a first metal atom and a second metal atom are adjacent to each other. Since the first metal atom and the second metal atom are adjacent to each other, the conversion efficiency of the intermetallic compound can increase.

It is possible to confirm whether the intermetallic compound contains the crystal lattice in which the first metal atom and the second metal atom are adjacent to each other, for example, by the following methods. First, the composition ratio of the intermetallic compound is analyzed, for example, by using an electron probe microanalyzer (EPMA), and then, the content ratio (atomic %) of the detected atoms in the intermetallic compound is determined by the ZAF method.

Similarly, an X-ray diffraction (XRD) measurement is performed on the intermetallic compound to obtain an X-ray diffraction spectrum. It is possible to determine whether the intermetallic compound contains the crystal lattice in which the first metal atom and the second metal atom are adjacent to each other, by identifying the intermetallic compound using, for example, an Inorganic Crystal Structure Database (ICSD), based on atom types and compositional ratios obtained by EPMA and the X-ray diffraction spectrum obtained by XRD measurement.

At least a part of the crystal lattice in the intermetallic compound of the present embodiment is preferably a hexagonal close-packed structure. If at least a part of the crystal lattice in the intermetallic compound is a hexagonal close-packed structure, the conversion efficiency of the intermetallic compound can be further improved.

The volume fraction of the hexagonal close-packed structure to the total volume of the intermetallic compound of the present embodiment is preferably 50% or more. If the volume fraction of the hexagonal close-packed structure is 50% or more, the conversion efficiency of the intermetallic compound can be further improved. The volume fraction of the hexagonal close-packed structure is preferably 60% or more, and more preferably 80% or more. The upper limit of the volume fraction of the hexagonal close-packed structure is not particularly limited, but is, for example, 100%.

When the intermetallic compound of the present embodiment contains a multi-coordinating atom, distortion may occur in the crystal lattice. In the intermetallic compound of the present embodiment, as long as the first metal atom and the second metal atom are adjacent to each other, distortion may exist in the crystal lattice.

To confirm that that at least a part of the crystal lattice in the intermetallic compound has a hexagonal close-packed structure, the crystal lattice can be identified from the composition ratio and X-ray diffraction spectrum of the intermetallic compound measured by the above-mentioned method using the Inorganic Crystal Structure Database (ICSD). The volume fraction occupied by the hexagonal close-packed structure can be obtained by creating a calibration curve based on the measurement results of XRD.

(Average Particle Size)

The average particle size of the intermetallic compound of the present embodiment is 500 μm or less. More preferably, the average particle size of the intermetallic compound is 100 μm or less. By having an average particle size of 500 μm or less, the contact area with $CO_2$ or CO can be increased, and the conversion efficiency in methanol synthesis is further improved, which is preferable. The average particle size of the intermetallic compound may be 1 μm or more.

The average particle size of the intermetallic compound is determined by observing the intermetallic compound with a field emission scanning electron microscope, selecting 50 intermetallic compounds from the obtained SEM image, and measuring the maximum value between any two points on the outline of each intermetallic compound. The average of the obtained values is regarded as the average particle size.

(BET Specific Surface Area)

The BET specific surface area of the intermetallic compound of the present embodiment is 1 $m^2$/g or more. More preferably, the BET specific surface area of the intermetallic compound is 2 $m^2$/g or more. By making the BET specific surface area 1 $m^2$/g or more, the contact area with $CO_2$ or CO can be increased, and the efficiency of methanol synthesis can be improved. The BET specific surface area of the intermetallic compound may be 1000 $m^2$/g or less.

The BET specific surface area of the intermetallic compound can be obtained by measurement using a specific surface area/pore distribution measuring device (e.g., BELSORP-mini II manufactured by MicrotracBEL Corporation) with nitrogen (purity 99.99995%) as the adsorption gas and the adsorption temperature set to the liquid nitrogen temperature (-196°C).

(Nitrides and Oxides of First and Second Metal Atoms)

The intermetallic compound of the present embodiment contains at least one of an oxide of at least one of the first metal atom and the second metal atom, and a nitride of at least one of the first metal atom and the second metal atom. The intermetallic compound of the present embodiment contains at least one of an oxide of at least one of the first metal atom and the second metal atom, and a nitride of at least one of the first metal atom and the second metal atom, which contributes to the stability of the crystal structure of the intermetallic compound in a high temperature region, and improves the high temperature resistance of the catalyst containing the intermetallic compound. The intermetallic compound of the present embodiment may contain only an oxide of at least one of the first metal atom and the second metal atom. The intermetallic compound of the present embodiment may also contain only a nitride of at least one of the first metal atom and the second metal atom.

Examples of the oxide of at least one of the first metal atom and the second metal atom include PdO, $PdO_2$, $RhO_2$, $Rh_2O_3$, $IrO_2$, $IrO_4$, $MoO_2$, $MoO_3$, and $Mo_2O_5$. Examples of the nitride of at least one of the first metal atom and the second metal atom include $PdN_2$, $RhN_2$, $IrN_2$, $Mo_2N$, MoN, and $MoN_2$.

To confirm that the sample contains at least one of an oxide of at least of one of the first metal atom and the second metal atom, and a nitride of at least one of the first metal atom and the second metal atom, the surface of the sample is subjected to elemental mapping using an EPMA. After elemental mapping, if N and the first metal atom or the second metal atom are detected in a predetermined ratio in the same region, it is determined that a nitride is present. After elemental mapping, if O and the first metal atom or the second metal atom are detected in a predetermined ratio in the same region, it is determined that an oxide is present . The predetermined ratio is determined according to the assumed compound.

<Method for Producing Intermetallic Compound>

Next, the method for producing an intermetallic compound of the present embodiment will be described with reference to FIG. 1. FIG. 1 is a flow chart of the method for producing an intermetallic compound of the present embodiment. The method for producing an intermetallic compound of the present embodiment is a method for producing an intermetallic compound containing a first metal atom and a second metal atom, the first metal atom being one or more selected from the group consisting of Pd, Rh, and Ir, and the second metal atom being Mo. The method for producing an intermetallic compound of the present embodiment includes a first step S1 in which a mixture, obtained by mixing a first compound containing a first metal atom and a second compound containing a second metal atom, is dehydrated to obtain a dehydrated-product, and then the dehydrated-product is carbonized to obtain a carbide; a second step S2 in which the carbide, obtained in the first step S1, is calcined under air to obtain a calcined-product; and a third step S3 in which the calcined-product, obtained in the second step S2, is subjected to a nitridation treatment in an $NH_3$ atmosphere. Each step will be described below.

(First Step S1)

In the first step S1, a first compound containing a first metal atom and a second compound containing a second metal atom are mixed together to obtain a mixture, which is then dehydrated, and the resulting dehydrated-product is carbonized.

(First Compound)

The first compound is a compound containing a first metal atom. The first metal atom is one or more selected from the group consisting of Pd, Rh, and Ir. The first compound is not particularly limited as long as it contains one or more selected from the group consisting of Pd, Rh, and Ir and is soluble in a solvent. Examples of the first compound include palladium acetate ($Pd(CH_3COO)_2$), rhodium acetate ($Rh_2(CH_3COO)_4$), and iridium acetate ($Ir(CH_3COO)_n$).

(Second Compound)

The second compound is a compound containing a second metal atom. The second metal atom is Mo. The second compound is not particularly limited as long as it contains Mo and is soluble in a solvent. Examples of the second compound include ammonium molybdate (VI) tetrahydrate ($(NH_4)_6Mo_7O_{24} \cdot 4H_2O$).

(Combination Ratio of First Compound and Second Compound)

The molar fraction of the first compound to the total moles of the first compound and the second compound is preferably 5 mol% or more and 95 mol% or less. More preferably, the molar fraction of the first compound is 10 mol% or more and 90 mol% or less. Further preferably, the molar fraction of the moles of the first compound is 10 mol% or more and 52 mol% or less. By having the molar fraction of the first compound to the total moles of the first compound and the second compound be 5 mol% or more and 95 mol% or less, the proportion of crystal lattices where the first metal atom and the second metal atom are adjacent to each other is increased, and the conversion efficiency in the methanol synthesis from $CO_2$ or CO of the intermetallic compound can be improved.

In the first step S1, the first compound and the second compound are mixed to obtain a mixture. The method for mixing the first compound and the second compound is not particularly limited. It is preferable to mix the first compound and the second compound in the presence of citric acid or polyvinylpyrrolidone. Mixing the first compound and the second compound in the presence of citric acid or polyvinylpyrrolidone may be performed, for example, by dissolving the first compound, the second compound, and citric acid or polyvinylpyrrolidone in a 6% nitric acid aqueous solution and mixing them. The solvent is not particularly limited as long as it can dissolve the first compound and the second compound, and water or an organic solvent such as methanol can be used. The acid is also not particularly limited as long as it is soluble in the solvent used for mixing, and nitric acid, sulfuric acid, hydrochloric acid, etc. can be used. The amount of citric acid or polyvinylpyrrolidone added is preferably at least twice the molar equivalent of the total molar number of the first metal and the second metal contained in the first compound and the second compound. By adding citric acid or polyvinylpyrrolidone in an amount of at least twice the molar equivalent of the total metal content of the first compound and the second compound, the uniformity of the material is easily maintained, and an intermetallic compound with higher conversion efficiency can be obtained in the synthesis of methanol from $CO_2$ or CO.

Next, the mixture obtained by mixing the first compound and the second compound is dehydrated to obtain a dehydrated-product. The method for dehydrating the mixture obtained by mixing the first compound and the second compound is not particularly limited. Here, "dehydration" means the general removal of water, and means not only drying the mixture but also removing water as a solvent. For example, when the mixture is a 6% nitric acid aqueous solution in which the first compound, the second compound, and citric acid or polyvinylpyrrolidone are dissolved, the aqueous solution is heated at a predetermined temperature (e.g., 80°C or higher) using a mantle heater or an oven, and the aqueous solution is reacted until the water in the aqueous solution is eliminated or until a gel is formed, to obtain a dehydrated-product.

After obtaining the dehydrated-product, the obtained dehydrated-product is carbonized to obtain a carbide. The method for carbonizing the dehydrated-product is not particularly limited. For example, the obtained dehydrated-product is heated to a predetermined temperature (e.g., 200°C or higher) to obtain a carbide. The heating time for obtaining the carbide is not particularly limited, but is, for example, 30 minutes or more.

(Second Step S2)

In the second step S2, the carbide obtained in the first step S1 is calcined under air to obtain a calcined-product. The method for calcining the carbide is not particularly limited. For example, the carbide obtained in the first step S1 is heated in a temperature range of 450°C to 600°C for 1 hour or more to obtain a calcined-product. The calcined-product is, for example, an oxide of the first metal atom and an oxide of the second metal atom.

(Third Step S3)

In the third step S3, the calcined-product obtained in the second step S2 is subjected to a nitridation treatment in an $NH_3$ atmosphere. This makes it possible to obtain the intermetallic compound of the present embodiment. The method for nitridation treatment in an $NH_3$ atmosphere is not particularly limited. For example, the calcined-product obtained in the second step S2 is heated under $NH_3$ at a nitridation treatment temperature for 6 hours or more. The nitridation treatment temperature is, for example, a temperature in the range of 600°C to 1000°C.

<Catalyst>

Next, a catalyst containing the intermetallic compound of the present embodiment will be described. The catalyst according to the present embodiment may contain the intermetallic compound of the present embodiment and a support that carries the intermetallic compound. The support may be omitted, and the intermetallic compound of the present embodiment may be used alone as a catalyst.

(Support)

The support is not particularly limited, and any known support can be used as long as it can support the intermetallic compound of the present embodiment and does not inhibit the desired chemical reaction such as the hydrogenation reaction of carbon dioxide and the hydrogenation reaction of carbon monoxide. For example, inorganic oxide supports such as silicon oxide (silica), zinc oxide, aluminum oxide (alumina), magnesium oxide (magnesia), indium oxide, calcium oxide, zirconium oxide (zirconia), titanium oxide (titania), hafnium oxide, barium oxide, cerium oxide (ceria), and other oxides of two or more metals such as perovskite type compounds or mayenite type compounds; nitride supports such as $Ta_3N_5$, BN, and $Si_3N_4$; carbon supports such as activated carbon and silicon carbide; and composites of two or more supports selected from the above supports.

The method for carrying the intermetallic compound of the present embodiment on the support can be a known method. For example, the intermetallic compound of the present embodiment and the support may be physically mixed, or the support may be coated with the intermetallic compound of the present embodiment. Alternatively, the first compound, the second compound, and citric acid or polyvinylpyrrolidone may be mixed with the support, and then, the above-mentioned first step S1 to third step S3 may be carried out to produce the support that carries the intermetallic compound.

<Method for Producing Methanol>

In the method for producing methanol according to the present embodiment, hydrogen and at least one of carbon monoxide and carbon dioxide are brought into contact with the intermetallic compound of the present embodiment. This makes it possible to obtain methanol. In the method for producing methanol according to the present embodiment, only carbon monoxide may be used, only carbon dioxide may be used, or a mixed gas of carbon monoxide and carbon dioxide may be used. The synthesis of methanol by the reaction of hydrogen and carbon monoxide is as shown in the following formula (1). The synthesis of methanol by the reaction of hydrogen and carbon dioxide is as shown in the following formula (2).

$$CO + 2H_2 \rightarrow CH_3OH... \qquad (1)$$

$$CO_2 + 3H_2 \rightarrow CH_3OH + H_2O... \qquad (2)$$

The method for bringing hydrogen and at least one of carbon monoxide and carbon dioxide into contact with the intermetallic compound of the present embodiment is not particularly limited. For example, a fixed bed flow type reactor may be used, and hydrogen and at least one of carbon dioxide or carbon monoxide may be introduced in accordance with the above reaction formula to cause a reaction. Hydrogen and at least one of carbon dioxide or carbon monoxide may be introduced into the reactor together with an inert gas. The inert gas used is, for example, Ar.

The reaction temperature between hydrogen and at least one of carbon monoxide and carbon dioxide is, for example, in the range from room temperature (e.g., 25°C) to 160°C. By using the intermetallic compound of the present embodiment, methanol can be synthesized at low temperatures.

The pressure during the reaction of hydrogen with at least one of carbon monoxide and carbon dioxide is in the range of atmospheric pressure (e.g., 0.1 MPa) to 2.0 MPa. By increasing the pressure, the conversion efficiency of the intermetallic compound in the synthesis of methanol from $CO_2$ or CO can be further improved.

<Method for Producing Carbon Monoxide>

In the method for producing carbon monoxide according to the present embodiment, hydrogen and carbon dioxide are brought into contact with the intermetallic compound of the present embodiment. The synthesis of carbon monoxide by the reaction of hydrogen and carbon dioxide is as shown in the following formula (3).

$$CO_2 + H_2 \rightarrow CO + H_2O... \qquad (3)$$

The method for bringing hydrogen and carbon dioxide into contact with the intermetallic compound of the present embodiment is not particularly limited. For example, a fixed-bed flow-type reactor may be used, and carbon dioxide and hydrogen may be introduced into the reactor in accordance with the reaction formula (3) above to cause the reaction. Carbon dioxide and hydrogen may be introduced into the reactor together with an inert gas. The inert gas used may be, for example, Ar.

The reaction temperature between hydrogen and carbon dioxide is, for example, in the range from room temperature (for example, 25°C) to 160°C. By using the intermetallic compound of the present embodiment, carbon monoxide can be synthesized at low temperatures.

The pressure during the reaction of hydrogen and carbon dioxide is from atmospheric pressure (for example, 0.1 MPa) to 2.0 MPa. By increasing the pressure, the conversion efficiency can be further improved in the synthesis of carbon monoxide from $CO_2$.

The intermetallic compound, the method for producing the intermetallic compound, the catalyst, the method for producing methanol, and the method for producing carbon monoxide according to the present embodiment have been described above. The intermetallic compound of the present embodiment can undergo a reaction at low temperatures, and furthermore has high conversion efficiency, and can be used repeatedly and stably. Therefore, by using the intermetallic compound of the present embodiment as a catalyst, methanol and carbon monoxide can be efficiently produced at room temperature.

The technical scope of the present invention is not limited to the above-described embodiment, and various modifications can be made without departing from the spirit of the present invention. In addition, the components in the above-described embodiment can be appropriately replaced with well-known components, and can also be appropriately combined with well-known components, without departing from the spirit of the present invention.

EXAMPLES

Next, an embodiment of the present invention will be described, but the conditions in the embodiment are merely an example of conditions adopted to confirm the feasibility and effects of the present invention, and the present invention is not limited to this example of conditions. Various conditions can be adopted in the present invention as long as they do not deviate from the gist of the present invention and achieve the object of the present invention.

(Example 1)

$(NH_4)_6Mo_7O_{24}\cdot4H_2O$ (manufactured by Aldrich) and $Pd(CH_3COO)_2$ (manufactured by Tokyo Chemical Industry Co., Ltd.) were used as raw materials (the metal molar ratio in the raw materials was set to Pd:Mo = 5mol%:95mol%), and the raw materials were stirred in a 6% aqueous nitric acid solution together with 2 times the equivalent of citric acid (manufactured by Kanto Chemical Co., Ltd.) with respect to the total amount of the metal sources. This aqueous solution was reacted at 80°C using a mantle heater until all water was removed to obtain a dehydrated-product. The obtained dehydrated-product was heated to 200°C to obtain a carbide. The obtained carbide was then calcined in air at 500°C for 2 hours to obtain a calcined-product (oxide precursor ($MoO_3$+PdO)). The obtained precursor was heated at 700°C for 12 hours under $NH_3$ flow to synthesize the intermetallic compound (hcp-PdMo/$Mo_2N$(Pd=5wt%(5mol%))) which is the catalyst of Example 1. The obtained hcp-PdMo/$Mo_2N$(Pd=5wt%(5mol%)) was in powder form (average particle size: 100 μm or less).

Example 2

An intermetallic compound (hcp-PdMo) of Example 2 was obtained under the same conditions as Example 1, except that $(NH_4)_6Mo_7O_{24}\cdot4H_2O$ (manufactured by Aldrich Co., Ltd.) and $Pd(CH_3COO)_2$ (manufactured by Tokyo Chemical Industry Co., Ltd.) were used as raw materials and the metal molar ratio in the raw materials was set to Pd:Mo = 52 mol% : 48 mol%. The catalyst of Example 2 obtained was in powder form (average particle size: 100 μm or less).

Example 3

An intermetallic compound serving as the catalyst of Example 3 was obtained under the same conditions as in Example 1, except that $(NH_4)_6Mo_7O_{24}\cdot4H_2O$ ( manufactured by Aldrich) and $Pd(CH_3COO)_2$ (manufactured by Tokyo Chemical Industry Co., Ltd.) were used as raw materials and the metal molar ratio in the raw materials was set to Pd:Mo=1 mol% : 99 mol%). The obtained catalyst of Example 3 was in powder form (average particle size: 100 μm or less).

Example 4

An intermetallic compound serving as the catalyst of Example 4 was obtained under the same conditions as in Example 1, except that $(NH_4)_6Mo_7O_{24}\cdot4H_2O$ (manufactured by Aldrich ) and $Pd(CH_3COO)_2$ ( manufactured by Tokyo Chemical Industry Co., Ltd.) were used as raw materials and the metal molar ratio in the raw materials was set to Pd:Mo=10 mol%:90 mol%). The obtained catalyst of Example 4 was in powder form (average particle size: 100 μm or less).

Example 5

$(NH_4)_6Mo_7O_{24}\cdot4H_2O$ (manufactured by Aldrich) and $Rh_2(CH_3COO)_4$ were both used, and the metal molar ratio in the raw materials was set to Rh:Mo=52mol% :48mol%, and the raw materials are stirred in a 6 % aqueous nitric acid solution together with citric acid (manufactured by Kanto Chemical Co., Ltd.) in an amount twice the amount of the total metal source. This aqueous solution is reacted at 80°C using a mantle heater until all water is removed to obtain a dehydrated-product. The obtained dehydrated-product is heated to 200°C to obtain a carbide. The obtained carbide is then calcined in air at 500°C for 2 hours to obtain a calcined-product (oxide precursor). The obtained precursor is heated at 700°C for 12 hours under an $NH_3$ stream to synthesize the intermetallic compound of Example 5.

(Example 6)

Both $(NH_4)_6Mo_7O_{24} \cdot 4H_2O$ (manufactured by Aldrich) and $Ir(CH_3COO)_n$) were used as raw materials, and the metal molar ratio in the raw materials was set to Ir:Mo=52mol%:48mol%). The raw materials are stirred in a 6% aqueous nitric acid solution together with citric acid (manufactured by Kanto Chemical Co., Ltd.) in an amount twice the amount of the total metal source. This aqueous solution is reacted at 80°C using a mantle heater until all water is removed to obtain a dehydrated-product. The obtained dehydrated-product is heated to 200°C to obtain a carbide. The obtained carbide is then calcined in air at 500°C for 2 hours to obtain a calcined-product (oxide precursor). The obtained precursor is heated at 700°C for 12 hours under a $NH_3$ stream to synthesize the intermetallic compound of Example 6.

(Comparative Example 1)

The catalyst was synthesized in the same manner as in Example 1, using only $(NH_4)_6Mo_7O_{24} \cdot 4H_2O$ (manufactured by Aldrich) as the raw material. The $Mo_2N$ synthesized by the above method was mixed with $Pd(acac)_2$ (manufactured by Aldrich). The resulting mixture was packed into a glass tube, and then heated at 300°C for 2 hours under a $H_2$ stream. As a result, a catalyst of Comparative Example 1, in which metal Pd particles were supported on $Mo_2N$, was obtained (Pd = 5 wt% (5 mol%)).

(Comparative Example 2)

The catalyst was synthesized using only $(NH_4)_6Mo_7O_{24} \cdot 4H_2O$ (manufactured by Aldrich Co.) as the raw material in the same manner as in Example 1. The obtained catalyst ($Mo_2N$) of Comparative Example 2 was in powder form.

(Comparative Example 3)

The catalyst of Comparative Example 3 was prepared by grinding pelletized $Cu/ZnO/Al_2O_3$ (manufactured by Alfa Aesar) into a powder using an agate mortar and pestle.

(Example 7)

$(NH_4)_6Mo_7O_{24} \cdot 4H_2O$ (manufactured by Aldrich) and $Pd(CH_3COO)_2$ (manufactured by Tokyo Chemical Industry Co., Ltd.) were used as raw materials (the metal molar ratio in the raw materials was set to Pd:Mo = 5mol%:95mol%), and the raw materials were stirred in a 6% aqueous nitric acid solution together with 2 times the equivalent amount of polyvinylpyrrolidone (manufactured by Fujifilm Wako Pure Chemical Industries, Ltd., Wako special grade (polymerization degree=K30)) with respect to the total amount of the metal sources. This aqueous solution was stirred at 90°C for 3 hours using an oil bath. Then, the aqueous solution was transferred to a drying pot, and the drying pot was placed in an oven and dried at 110°C for 8 hours until the contents in the drying pot formed a gel. In this way, a dehydrated-product was obtained that had been reacted until it became a gel. The obtained dehydrated-product was calcined in air at 500°C for 2 hours to obtain a calcined-product (oxide precursor ($MoO_3$+PdO)). The obtained oxide precursor was heated in an $NH_3$ stream at 700°C for 12 hours to synthesize an intermetallic compound (hcp-PdMo/$Mo_2N$(Pd=5mol%)) which is the catalyst of Example 7. The obtained intermetallic compound (hcp-PdMo/$Mo_2N$(Pd=5mol%)) which is the catalyst of Example 7 was in a powder form (average particle size: 100 μm or less).

(Example 8)

An intermetallic compound (hcp-PdMo) corresponding to the intermetallic compound of Example 7 was obtained under the same conditions as in Example 7, except that $Pd(acac)_2$ (manufactured by Aldrich ) was used instead of $Pd(CH_3COO)_2$ as raw material. The obtained intermetallic compound, which is the catalyst of Example 8 was in powder form (average particle size: 100μm or less).

(Example 9)

An intermetallic compound (hcp-PdMo) corresponding to the intermetallic compound of Example 7 was obtained under the same conditions as in Example 7, except that $Pd(NO_3)_2 \cdot 2H_2O$ (manufactured by Aldrich Co.) was used instead of $Pd(CH_3COO)_2$ as the raw material. The obtained intermetallic compound, which is the catalyst of Example 9, was in powder form (average particle size: 100 μm or less).

(Powder X-ray diffraction measurement)

The powder X-ray diffraction measurements of Examples 1 to 4 and Comparative Example 1 were carried out by the following method. An X-ray diffractometer (MiniFlex 600, manufactured by RIGAKU) was used, and CuKα was used as the X-ray source. The measurement temperature was room temperature (25°C), the measurement angle range was 20 to 70°, the scan speed was 0.06 seconds/step, and the scan step was 0.01°. The results obtained are shown in FIG. 2. X-ray diffraction measurements were also carried out in the same manner for Example 2 after the temperature-programmed desorption measurement described below. In addition, X-ray diffraction measurements were also carried out in the same manner for Example 2 after 10 days in the atmosphere and for Example 1 after the continuous reaction.

(Temperature-programmed Desorption Measurement)

The temperature-programmed desorption measurement in Example 2 was carried out using BELCAT II manufactured by MicrotracBEL Corporation. Specifically, the temperature-programmed desorption measurement was carried out using a 50 mg sample, heating the sample from room temperature (25°C) to 1000°C at a rate of 10°C/min in an Ar atmosphere, and then holding the sample at 1000°C for 1 hour. The gas species desorbed from the sample at this time was analyzed using a mass spectrometer attached to a catalyst analyzer (e.g., BELCAT II manufactured by MicrotracBEL Corporation). The obtained results are shown in FIG. 7.

(EPMA Analysis)

The EPMA analysis of Example 2 was carried out using a JXA-8530F manufactured by JEOL Ltd. First, elemental mapping of the catalyst of Example 2 was carried out. The obtained mapping image is shown in FIG. 6. The composition ratio of the catalyst of Example 2 was determined by performing composition analysis using the ZAF method at 50 random locations in the region where Pd and Mo were detected in the obtained mapping image, and the average value was taken as the composition ratio of Example 2. The obtained results are shown in Table 1.

(BET Specific Surface Area Measurement)

The BET specific surface area measurements of Examples 1 to 8 and Comparative Examples 1 to 3 were carried out using a specific surface area/pore distribution measuring device (BELSORP-mini II, manufactured by MicrotracBEL Corporation). Nitrogen gas (purity 99.99995% by volume) was used as the adsorption gas, and the adsorption temperature was set to the liquid nitrogen temperature (-196°C). The obtained results are shown in Tables 2 to 5.

(Evaluation of Effect of Catalysts on Reaction of $CO_2$ Under Atmospheric Pressure)

The effect of the catalyst on the reaction of carbon dioxide and hydrogen under atmospheric pressure (0.1 MPa) was evaluated. Specifically, carbon dioxide gas ($CO_2$) and hydrogen gas ($H_2$) were brought into contact with any one of the catalysts of Examples 1, 2, and 4 and Comparative Examples 1 to 3 under atmospheric pressure to synthesize methanol. 0.1 g of the catalyst was packed into a glass tube and used, and the synthesis reaction was carried out using a fixed-bed flow-type reactor as the reaction apparatus. Before starting the reaction, a pretreatment was carried out at 300°C for 2 hours under $H_2$ flow. During the reaction, the gas flow rates were as follows: $CO_2$:10mL/min, $H_2$:30mL/min, Ar:10mL/min, totaling 50 mL/min. The reaction was carried out at a reaction temperature of 100°C. An Agilent gas chromatograph 7890A was used to confirm the methanol synthesis. A DB-1 column (manufactured by Agilent) was used, and the column temperature was set in the range of 50 to 200°C. The flow path from the reaction apparatus to the gas chromatograph was heated to 90°C or higher using a tube heater. The results obtained are shown in Table 2.

(Evaluation of Effect of Catalysts on CO Reactions Under Atmospheric Pressure)

The effect of the catalyst on the reaction of carbon monoxide and hydrogen under atmospheric pressure was evaluated. Carbon monoxide gas (CO) and hydrogen gas ($H_2$) were brought into contact with any one of the catalysts of Examples 1 to 4 and Comparative Examples 1 to 3 to carry out a reaction to synthesize methanol. 0.1 g of each catalyst was packed into a glass tube and used, and the synthesis reaction was carried out using a fixed-bed flow-type reactor as the reaction apparatus. Before starting the reaction, a pretreatment was carried out for 2 hours at 300°C under a $H_2$ stream. During the reaction, the gas flow rates were as follows: CO:10mL/min, $H_2$:20mL/min, Ar:10mL/min, totaling 40 mL/min. The reaction was carried out at a reaction temperature of 140°C. The flow path from the reactor to the gas chromatograph was heated to 90°C or higher using a tube heater. The results are shown in Table 3.

(Evaluation of Effect of $Mo_2N$ on Reaction of CO Under Atmospheric Pressure )

The effect of $Mo_2N$ on the reaction of carbon monoxide and hydrogen under atmospheric pressure (0.1 MPa) was evaluated. Specifically, carbon monoxide gas (CO) and hydrogen gas ($H_2$) were brought into contact with the catalyst of Example 1 or Example 2 under atmospheric pressure to synthesize methanol. 0.1 g of the catalyst was packed into a glass tube, and the synthesis reaction was carried out using a fixed-bed flow-type reactor as the reaction apparatus. Before starting the reaction, a pretreatment was carried out under $H_2$ flow at 300°C for 2 hours. During the reaction, the gas flow rates were as follows: CO:10mL/min, $H_2$:20mL/min, Ar:10mL/min, totaling 40 mL/min. The reaction was carried out at a reaction temperature of 60°C to 180°C. An Agilent gas chromatograph 7890A was used to confirm the methanol synthesis. A DB-1 column (manufactured by Agilent) was used, and the column temperature was set in the range of 50 to 200°C. The flow path from the reaction apparatus to the gas chromatograph was heated to 90°C or higher using a tube heater. The results obtained are shown in FIG. 9.

(Evaluation of Effect of Pressurization on Reaction of $CO_2$)

The effect of pressurization on the reaction between carbon dioxide and hydrogen was evaluated. Specifically, carbon dioxide gas ($CO_2$) and hydrogen gas ($H_2$) were brought into contact with the catalyst of Example 1 under atmospheric pressure (0.1 MPa) or under pressure (0.9 MPa) to carry out a reaction to synthesize methanol. 0.1 g of the catalyst was packed into a glass tube, and the synthesis reaction was carried out using a fixed-bed flow-type reactor as the reaction apparatus. Before starting the reaction, a pretreatment was carried out for 2 hours at 300°C under a $H_2$ stream. During the reaction, the gas flow rates were as follows: $CO_2$:10mL/min, $H_2$:30mL/min, Ar: 10mL/min, totaling 50 mL/min. The reaction was carried out at a reaction temperature of 25°C to 80°C. An Agilent gas chromatograph 7890A was used to confirm the methanol synthesis. A DB-1 column (manufactured by Agilent) was used, and the column temperature was set in the range of 50°C to 200°C. The flow path from the reaction apparatus to the gas chromatograph was heated to 90°C or higher using a tube heater. The results obtained are shown in FIG.10.

(Long-Term Stability Evaluation)

Next, the long-term stability of the catalyst was evaluated. Specifically, carbon monoxide gas (CO) and hydrogen gas ($H_2$) were brought into contact with the catalyst of Example 1 under atmospheric pressure (0.1 MPa) to carry out a reaction for a long time to synthesize methanol. 0.1 g of the catalyst was packed into a glass tube, and a synthesis reaction was carried out using a fixed-bed flow-type reactor as the reaction apparatus. Before starting the reaction, a pretreatment was carried out for 2 hours at 300°C under $H_2$ flow. The flow rates of the gases during the reaction were $CO_2$:10mL/min, $H_2$:30mL/min, Ar:10mL/min, totaling 50 mL/min. The reaction temperature was 100°C, and the reaction was carried out for 0 to 100 hours. An Agilent gas chromatograph 7890A was used to confirm the methanol synthesis. A DB-1 column (manufactured by Agilent) was used, and the column temperature was set in the range of 50 to 200°C. The flow path from the reaction apparatus to the gas chromatograph was heated to 90°C or higher using a tube heater. The results obtained are shown in FIG. 11. In addition, X-ray diffraction measurement was performed on the catalyst of the present embodiment after the reaction was completed. The results obtained are shown in FIG. 12.

(Evaluation of Effect of Catalysts on Carbon Monoxide Synthesis Under Atmospheric Pressure)

The effect of the catalyst on the reaction of carbon dioxide and hydrogen under atmospheric pressure (0.1 MPa) was evaluated. Carbon dioxide gas ($CO_2$) and hydrogen gas ($H_2$) were brought into contact with any one of the catalysts of Example 1 and Comparative Examples 1 and 2 to carry out a reaction to synthesize carbon monoxide. 0.1 g of each catalyst was packed into a glass tube and used, and the synthesis reaction was carried out using a fixed-bed flow-type reactor as the reaction apparatus. Before starting the reaction, a pretreatment was carried out for 2 hours at 300°C under a $H_2$ stream. During the reaction, the gas flow rates were as follows: $CO_2$:10mL/min, $H_2$:30mL/min, Ar:10mL/min, totaling 50mL/min. The reaction was carried out at a reaction temperature of 140°C. The flow path from the reactor to the gas chromatograph was heated to 90°C or higher using a tube heater. The results obtained are shown in Table 4.

(Evaluation of Effect of Reaction Temperature on Carbon Monoxide Synthesis)

The effect of reaction temperature on carbon monoxide synthesis under atmospheric pressure (0.1 MPa) was evaluated. Specifically, carbon dioxide gas ($CO_2$) and hydrogen gas ($H_2$) were brought into contact with the catalyst of Example 1 or Comparative Example 1 under atmospheric pressure to synthesize carbon monoxide. 0.1 g of each catalyst was packed into a glass tube and used, and the synthesis reaction was carried out using a fixed-bed flow-type reactor as the reaction apparatus. Before starting the reaction, a pretreatment was carried out under $H_2$ stream at 300°C for 2 hours.

During the reaction, the gas flow rates were as follows: $CO_2$:10mL/min, $H_2$:30mL/min, Ar:10mL/min, totaling 50 mL/min. The reaction was carried out at a reaction temperature of 120 to 180°C. An Agilent gas chromatograph 7890A was used to confirm the synthesis of carbon monoxide. A DB-1 column (manufactured by Agilent) was used, and the column temperature was set in the range of 50 to 200°C. The flow path from the reaction apparatus to the gas chromatograph was heated to 90°C or higher using a tube heater. The results obtained are shown in FIG. 13.

FIG. 2 shows the X-ray diffraction spectra of Examples 1 to 4, Comparative Example 2, and $Mo_2N$ (ICSD No. 251625) in the Inorganic Crystal Structure Database (ICSD). The horizontal axis of FIG. 2 is 20 (deg.), and the vertical axis represents intensity. As shown in FIG. 2, the peak positions of Comparative Example 2 coincided with the peak positions of $Mo_2N$ in ICSD, so Comparative Example 2 was confirmed to be $Mo_2N$. The peak of $Mo_2N$ was confirmed in Examples 1, 3, and 4. When the Pd content was 1 wt%, the peak position of the observed catalyst was similar to that of $Mo_2N$ in ICSD. This is because the "new peaks" present between 42° and 44° and between 55° and 58° were small and overlapped with the large peaks of $Mo_2N$, and it is considered that the presence of the "new peaks" could not be clearly confirmed from the X-ray diffraction spectrum. On the other hand, when the Pd content was 5 wt% or more, it was clearly confirmed that "new peaks" appeared between 42° and 44° and between 55° and 58°. In particular, in Example 2, in which the Pd content was 52 wt%, the peaks derived from $Mo_2N$ disappeared and only the "new peaks" were observed.

FIG. 3 shows the X-ray diffraction spectra of Example 2 and PdMo having hexagonal close-packed structure (hcp-PdMo) obtained from the ICSD. The horizontal axis of FIG. 3 is 20 (deg.), and the vertical axis represents intensity. Since the blending ratio of Pd and Mo was approximately 1:1, and the peak position of the catalyst of Example 2 coincided with the peak position of hcp-PdMo in ICSD, it was confirmed that PdMo (hcp-PdMo) of Example 2 has a hexagonal close-packed structure. From the above, it was confirmed that at least Examples 1, 2, and 4 include a crystal lattice in which the first metal atom and the second metal atom are adjacent to each other.

FIG. 4 shows the X-ray diffraction spectra of Example 2 before and after the temperature-programmed desorption measurement, and Mo (No. 52267) and Pd (No. 52251) in ICSD. The horizontal axis of FIG. 4 is 2θ (deg.), and the vertical axis represents intensity. After the temperature-programmed desorption measurement, the intensity of the peak between 42° and 44° and the intensity of the peak between 55° and 58° decreased, and new peaks appear. The positions of these new peaks coincide with the peak positions of the elemental form of Mo and the elemental form of Pd in ICSD, so it is inferred that hcp-PdMo is decomposed into Pd and Mo by performing the temperature-programmed desorption measurement.

FIG. 5 shows the X-ray diffraction spectra of the catalyst of Example 2 immediately after preparation and the catalyst of Example 2 stored in the air for 10 days after preparation. The horizontal axis of FIG. 5 is 2θ (deg.), and the vertical axis represents intensity. As shown in FIG. 5, the X-ray diffraction spectrum of the catalyst of Example 2 did not change even after 10 days in the atmosphere. From the above, it was confirmed that the catalyst of Example 2 is stable in the atmosphere.

FIG. 6 shows an element mapping image of the catalyst of Example 2. The upper right figure is an element mapping image of Mo, and the lower left figure is an element mapping image of Pd. Since Mo and Pd overlap, it was confirmed that the catalyst of Example 2 is an intermetallic compound consisting of Pd and Mo. As shown in Table 1, N and O were present in a certain ratio in the catalyst of Example 2. From this result, the composition ratio of Example 2 was estimated to be $Pd_{0.6}Mo_{0.4}N_{0.1}O_{0.1}$.

The results of the temperature-programmed desorption measurement of the catalyst of Example 2 are shown in FIG. 7. The horizontal axis of FIG. 7 indicates the measurement time, the first vertical axis indicates the $N_2$ intensity, and the second vertical axis indicates the temperature. As shown in FIG. 7, it was confirmed that $N_2$ was desorbed by raising the temperature to 1000°C. After the temperature-programmed desorption measurement, as shown in FIG. 4, PdMo was separated into elemental form of Pd and elemental form of Mo, so it is presumed that the presence of nitrogen in PdMo improved the stability of the catalyst of Example 2.

Table 1

| | Pd (mol%) | Mo (mol%) | N (mol%) | O (mol%) |
|---|---|---|---|---|
| Example 2 | 48.2 | 36.1 | 6.9 | 8.9 |

Table 2 shows the particle size, BET specific surface area, and methanol synthesis rate when carbon dioxide gas is used for Examples 1, 2, and 4 and Comparative Examples 1 to 3. Examples 1, 2, and 4 in which the intermetallic compound of the present embodiment includes a crystal lattice in which Pd and Mo are adjacent to each other, and the X-ray diffraction spectrum before the temperature-programmed desorption measurement has a first peak between 42° and 44° and a second peak between 55° and 58° in terms of a diffraction angle 2θ, and the positions of the first peak and the second peak are different from the positions of the peaks of the elemental form of Pd and the elemental form of Mo, and after the temperature-programmed desorption measurement, the intensities of the first peak and the second peak are equal to or lower than those before the temperature-programmed desorption measurement, and the BET specific surface area is 1 $m^2/g$ or more showed high catalytic performance compared to Comparative Examples 1 to 3. Comparative Examples 1 and 2 in which Pd and Mo are not adjacent to each other could hardly synthesize methanol. Moreover, Comparative Example 3, which is a conventional catalyst, also did not provide a high conversion efficiency.

Table 3 shows the particle size, BET specific surface area, and methanol synthesis rate when carbon monoxide gas is used for Examples 1 to 4 and Comparative Examples 1 to 3. Examples 1 to 4 in which the intermetallic compound of the present embodiment includes a crystal lattice in which Pd and Mo are adjacent to each other, and the X-ray diffraction spectrum before the temperature-programmed desorption measurement has a first peak between 42° and 44° and a second peak between 55° and 58° in terms of a diffraction angle $2\theta$, and the positions of the first peak and the second peak are different from the positions of the peaks of the elemental form of Pd and the elemental form of Mo, and after the temperature-programmed desorption measurement, the intensities of the first peak and the second peak are equal to or lower than those before the temperature-programmed desorption measurement, and the BET specific surface area is 1 $m^2/g$ or more showed high catalytic performance compared to Comparative Examples 1 to 3. Comparative Examples 1 and 2 in which Pd and Mo are not adjacent to each other could hardly synthesize methanol. Moreover, Comparative Example 3, which is a conventional catalyst, also did not provide a high conversion efficiency.

Table 2

| | Catalyst | BET Specific Surface Area ($m^2/g$) | Methanol Synthesis Rate ($CO_2$ Hydrogenation) ($\mu mol \cdot g^{-1} \cdot h^{-1}$) |
|---|---|---|---|
| Example 1 | hcp-PdMo/$Mo_2N$ (Pd=5 mol%) | 12.9 | 14.8 |
| Example 2 | hcp-PdMo | 2.6 | 25.2 |
| Example 4 | hcp-PdMo/$Mo_2N$ (Pd=10 mol%) | 13.9 | 23.8 |
| Comparative Example 1 | Pd/$Mo_2N$ (Pd=5 mol%) | 25.2 | 0 |
| Comparative Example 2 | $Mo_2N$ | 19.6 | 0 |
| Comparative Example 3 | Cu/ZnO/$Al_2O_3$ | 86.5 | 8.3 |

Table 3

| | Catalysts | BET Specific Surface Area ($m^2/g$) | Methanol Synthesis Rate (CO Hydrogenation) ($\mu mol \cdot g^{-1} \cdot h^{-1}$) |
|---|---|---|---|
| Example 1 | hep-PdMo/$Mo_2N$ (Pd=5 mol%) | 12.9 | 160.9 |
| Example 2 | hcp-PdMo | 2.6 | 175.6 |
| Example 3 | hcp-PdMo/$Mo_2N$ (Pd=1 mol%) | 17.4 | 33.9 |
| Example 4 | hcp-PdMo/$Mo_2N$ (Pd=10 mol%) | 13.9 | 230.6 |
| Comparative Example 1 | Pd/$Mo_2N$ (Pd=5 mol%) | 25 | 3.4 |
| Comparative Example 2 | $Mo_2N$ | 19.6 | 0 |
| Comparative Example 3 | Cu/ZnO/$Al_2O_3$ | 86.5 | 9.1 |

FIG. 8 shows the relationship between the Pd content in the catalyst and the methanol synthesis rate when carbon dioxide is used. The horizontal axis of FIG. 8 is the Amount of Pd (mol%), and the vertical axis represents the methanol synthesis rate ($\mu mol \cdot g^{-1} \cdot h^{-1}$). The methanol synthesis rate increased significantly up to an amount of Pd of 10 mol%, but did not increase significantly thereafter, even when the amount of Pd exceeded 50 mol%. Therefore, it was confirmed that a Pd content of 10 mol% or more is preferable.

FIG. 9 shows the relationship between reaction temperature and methanol synthesis rate in the catalysts of Example 1 and Example 2. The horizontal axis of FIG. 9 is reaction temperature (°C), and the vertical axis represents methanol synthesis rate ($\mu mol \cdot g^{-1} \cdot h^{-1}$). As shown in FIG. 9, it was confirmed that the methanol synthesis rate ($\mu mol \cdot g^{-1} \cdot h^{-1}$) increases as the reaction temperature increases. Moreover, regardless of the presence or absence of $Mo_2N$, Example 1 (hcp-PdMo/$Mo_2N$) and Example 2 (hcp-PdMo) show the same level of methanol synthesis rate, and it was confirmed that the presence of $Mo_2N$ does not have a particularly adverse effect on the catalyst performance.

FIG. 10 shows the relationship between reaction temperature and methanol synthesis rate when the pressurization conditions are changed. The horizontal axis of FIG. 10 is reaction temperature (°C), and the vertical axis represents methanol synthesis rate ($\mu mol \cdot g^{-1} \cdot h^{-1}$). As shown in FIG. 10, it was confirmed that the methanol synthesis rate ($\mu mol \cdot g^{-1} \cdot h^{-1}$) of synthesizing methanol from carbon dioxide increases as the reaction temperature increases. It was also confirmed that pressurization further improves the reaction rate. From the above, it was confirmed that the catalyst according to the present embodiment allows the methanol synthesis reaction to proceed even at room temperature by pressurization.

FIG. 11 shows the relationship between the reaction time and the methanol synthesis rate in the catalyst of Example 1. The horizontal axis of FIG. 11 is the reaction time (h), and the vertical axis represents the methanol synthesis rate ($\mu mol \cdot g^{-1} \cdot h^{-1}$). As shown in FIG. 11, the catalyst of Example 1 did not deteriorate in the methanol synthesis rate ($\mu mol \cdot g^{-1} \cdot h^{-1}$) even when it synthesized methanol continuously for 100 hours. FIG. 12 shows the X-ray diffraction spectrum of the catalyst according to the present embodiment before and after the continuous reaction. The horizontal axis of FIG. 12 is $2\theta$ (deg.), and the vertical axis represents intensity. As shown in FIG. 12, since there is no change in the X-ray diffraction spectrum of the catalyst according to the present embodiment before and after the continuous reaction, it was confirmed that the stability of the catalyst according to the present embodiment is high. From the above, it was confirmed that the catalyst containing the intermetallic compound of the present embodiment has a high conversion efficiency and can be repeatedly and stably used in the methanol synthesis from $CO_2$ or CO.

Table 4 shows the BET specific surface areas (specific surface areas of catalyst) and the carbon monoxide synthesis rate when carbon dioxide gas is used, for Example 1, Comparative Example 1, and Comparative Example 2. As shown in Table 4, Example 1 in which the catalyst includes a crystal lattice in which Pd and Mo are adjacent to each other, and has a first peak between 42° and 44° and a second peak between 55° and 58° in terms of a diffraction angle 2θ; the positions of the first and second peaks are different from the positions of the peaks of the elemental form of Pd and the elemental form of Mo; after the temperature-programmed desorption measurement, the intensities of the first and second peaks are equal to or lower than those before the temperature-programmed desorption measurement; and the catalyst of Example 1 has a BET specific surface area of 1 $m^2/g$ or more exhibited higher catalytic performance than Comparative Examples 1 and 2.

Table 4

| | Catalysts | BET Specific Surface Area ($m^2/g$) | CO Synthesis Rate ($\mu mol \cdot g^{-1} \cdot h^{-1}$) |
|---|---|---|---|
| Example 1 | hcp-PdMo/$Mo_2N$ (Pd=5 mol%) | 12.9 | 432.1 |
| Comparative Example 1 | Pd/$Mo_2N$ (Pd=5 mol%) | 25.2 | 82.2 |
| Comparative Example 2 | $Mo_2N$ | 19.6 | 0 |

FIG. 13 shows the relationship between reaction temperature and carbon monoxide synthesis rate in the catalysts of Example 1 and Comparative Example 1. The horizontal axis of FIG. 13 is reaction temperature (°C), and the vertical axis represents carbon monoxide synthesis rate ($\mu mol \cdot g^{-1} \cdot h^{-1}$). As shown in FIG. 13, it was confirmed that the carbon monoxide synthesis rate ($\mu mol \cdot g^{-1} \cdot h^{-1}$) improves as the reaction temperature increased. From the above, it was confirmed that the catalyst according to the present embodiment also exhibits high catalytic performance in carbon monoxide synthesis.

(Evaluation of Effect of Catalysts on Reaction of $CO_2$ Under Atmospheric Pressure, Part 2)

The effect of the catalyst on the reaction between carbon dioxide and hydrogen under atmospheric pressure (0.1 MPa) was evaluated. Specifically, a reaction to synthesize methanol was carried out by bringing carbon dioxide gas ($CO_2$) and hydrogen gas ($H_2$) into contact with any one of the catalysts of Examples 1 and 7 to 9 under atmospheric pressure. After 0.1 g of the catalyst was packed into a glass tube, a synthesis reaction was carried out using a fixed-bed flow-type reactor equipped with the obtained catalyst-filled glass tube as a reaction apparatus. Before starting the reaction, a pretreatment was carried out at 300°C for 2 hours under $H_2$ flow. During the reaction, the gas flow rates were as follows: $CO_2$:10mL/min, $H_2$:30mL/min, Ar:10mL/min, totaling 50 mL/min. The reaction was carried out at a reaction temperature of 100°C. An Agilent gas chromatograph 7890A was used to confirm the methanol synthesis. A DB-1 column (manufactured by Agilent) was used, and the column temperature was set in the range of 50 to 200°C. The flow path from the reaction apparatus to the gas chromatograph was heated to 90°C or higher using a tube heater. The results obtained are shown in Table 5.

[Table 5]

| | Catalyst | Pd Raw Material | Chelating Agent | BET Specific Surface Area ($m^2/g$) | Methanol Synthesis Rate ($CO_2$ Hydrogenation) ($\mu mol \cdot g^{-1} \cdot h^{-1}$) |
|---|---|---|---|---|---|
| Example 1 | hcp-PdMo/$Mo_2N$ (Pd=5 mol%) | $Pd(OAc)_2$ | Citric Acid | 12.9 | 52 |
| Example 7 | hcp-PdMo/$Mo_2N$ (Pd=5 mol%) | $Pd(OAc)_2$ | Polyvinylpyrrolidone | 11.2 | 59.7 |
| Example 8 | hcp-PdMo/$Mo_2N$ (Pd=5 mol%) | $Pd(acac)_2$ | Polyvinylpyrrolidone | 41.6 | 79.8 |
| Example 9 | hcp-PdMo/$Mo_2N$ (Pd=5 mol%) | $Pd(NO_3)_2 \cdot 2H_2O$ | Polyvinylpyrrolidone | 11.4 | 47.7 |

(Discussion)

Table 5 shows the BET specific surface areas of Example 1 and Examples 7 to 9, and the methanol synthesis rates when carbon dioxide gas was used. The catalysts of Example 7 and Example 9, which have similar specific surface areas, showed similar methanol synthesis rates at a reaction temperature of 160°C, while the catalyst of Example 8, which had a high specific surface area, showed a reaction rate that was about 30% higher. It is believed that the reaction rate increased as a result of an increase in the number of active sites, which was achieved by highly dispersing and supporting the PdMo intermetallic compound through appropriate selection of the Pd-source and the chelating agent.

(Evaluation of Effect of Reaction Temperature on Reaction between Carbon Dioxide and Hydrogen)

The effect of the reaction temperature on the reaction between carbon dioxide and hydrogen under atmospheric pressure (0.1 MPa) was evaluated. Specifically, a reaction to synthesize methanol was carried out by bringing carbon dioxide gas ($CO_2$ ) and hydrogen gas ($H_2$) into contact with any one of the catalysts of Examples 1 and 7 to 9 under atmospheric pressure. After 0.1 g of the catalyst was packed into a glass tube, a synthesis reaction was carried out using a fixed-bed flow-type reactor equipped with the obtained catalyst-filled glass tube as a reaction apparatus. Before starting the reaction, a pretreatment was carried out at 300°C for 2 hours under $H_2$ gas flow. During the reaction, the gas flow rates were as follows: $CO_2$:10mL/min, $H_2$:30mL/min, Ar:10mL/min, totaling 50mL/min. The reaction was carried out at a reaction temperature of 100°C to 200°C. An Agilent gas chromatograph 7890A was used to confirm the methanol synthesis. A DB-1 column (manufactured by Agilent) was used, and the column temperature was set in the range of 50 to 200°C. The flow path from the reaction apparatus to the gas chromatograph was heated to 90°C or higher using a tube heater. The results obtained are shown in FIG. 14.

(Discussion)

FIG. 14 shows that all of the PdMo catalysts synthesized by changing the Pd source and the chelating agent operated at the same reaction temperature as in Example 1, and showed the same or higher catalytic activity than those in Example 1. From the results of X-ray diffraction, peaks derived from the PdMo intermetallic compound were observed in all samples, suggesting that the PdMo intermetallic compound contributes to the high methanol synthesis activity at low temperatures.

(Evaluation of Relationship between Selectivity to Methanol and Reaction Temperature in Reaction of Carbon Dioxide And Hydrogen)

Relationship between the selectivity to methanol in the reaction of carbon dioxide and hydrogen under atmospheric pressure (0.1 MPa) and the reaction temperature was evaluated. Specifically, a reaction to synthesize methanol was carried out by bringing carbon dioxide gas ($CO_2$) and hydrogen gas ($H_2$) into contact with the catalyst of Example 1 or Example 8 under atmospheric pressure. After 0.1 g of the catalyst was packed into a glass tube, a synthesis reaction was carried out using a fixed-bed flow-type reactor equipped with the obtained catalyst-filled glass tube as a reaction apparatus. Before starting the reaction, a pretreatment was carried out at 300°C for 2 hours under a $H_2$ stream. During

the reaction, the gas flow rates were as follows: $CO_2$:10mL/min, $H_2$:30mL/min, Ar:10mL/min, totaling 50 mL/min. The reaction was carried out at a reaction temperature of 50°C to 250°C. An Agilent gas chromatograph 7890A was used to confirm the methanol synthesis. The column used was an Agilent DB-1, and the column temperature was set in the range of 50 to 200°C. The flow path from the reactor to the gas chromatograph was heated to 90°C or higher using a tube heater. The selectivity to methanol at each reaction temperature was measured by the following method. The results are shown in FIGS. 15 and 16.

<Method for Evaluating Selectivity to Methanol>

Selectivity to methanol was measured by measuring the concentrations of CO, MeOH, and $CH_4$ using a gas chromatograph. The selectivity to methanol (%) was calculated using the following formula:

$$\text{Selectivity to methanol (\%)} = \text{MeOH}/(\text{MeOH}+\text{CO}+\text{CH}_4)$$

(Discussion)

The relationship between the methanol selectivity and the reaction temperature in the catalyst of Example 1 or Example 8 is shown. The horizontal axis of FIGS. 15 and 16 is the reaction temperature (°C), and the vertical axis represents the selectivity (%) of each product. As shown in FIG. 15, the catalyst of Example 1 showed a methanol selectivity of approximately 100% at a reaction temperature of 60°C. As the reaction temperature increased, the methanol selectivity decreased, but the CO selectivity tended to improve. On the other hand, as shown in FIG. 16, the catalyst of Example 8 showed a high methanol selectivity of 80% or more up to a reaction temperature of about 120°C. When the reaction temperature reached 140°C or higher, the CO selectivity improved significantly. The reason why the catalyst of Example 8 showed a high methanol selectivity is not fully understood at this time, but it is believed that the PdMo nanoparticle is the main factor of the high methanol selectivity.

## Claims

1. An intermetallic compound, comprising a first metal atom and a second metal atom and having a crystal lattice in which the first metal atom and the second metal atom are adjacent to each other,

    wherein the first metal atom is at least one selected from the group consisting of Pd, Rh, and Ir,
    the second metal atom is Mo,
    the X-ray diffraction spectrum before the temperature-programmed desorption measurement has a first peak between 42° and 44° and a second peak between 55° and 58° in terms of a diffraction angle 2$\theta$,
    a position of the first peak and a position of the second peak are different from a position of a peak of the elemental form of the first metal atom and a position of a peak of the elemental form of the second metal atom,
    after the temperature-programmed desorption measurement, the intensity of the first peak and the intensity of the second peak are equal to or lower than those before the temperature-programmed desorption measurement, and
    the intermetallic compound has a BET specific surface area of 1 $m^2$/g or more.

2. The intermetallic compound according to claim 1, wherein at least a portion of the crystal lattice has a hexagonal close-packed structure.

3. The intermetallic compound according to claim 2, wherein a volume fraction of the hexagonal close-packed structure with respect to a total volume of the intermetallic compound is 50% or more.

4. The intermetallic compound according to claim 1, wherein a molar fraction of the first metal atoms with respect to total moles of the first metal atoms and the second metal atoms contained in the intermetallic compound is 5 mol% or more and 95 mol% or less.

5. The intermetallic compound according to claim 1, wherein a molar fraction of the first metal atoms with respect to total moles of the first metal atoms and the second metal atoms contained in the intermetallic compound is 10 mol% or more and 90 mol% or less.

6. The intermetallic compound according to claim 1, wherein a molar ratio of the first metal atoms to the second metal atoms is from 30:70 to 70:30.

7. The intermetallic compound according to claim 6, wherein a molar ratio of the first metal atoms to the second metal atoms is from 40:60 to 65:35.

8. The intermetallic compound according to claim 7, wherein a molar ratio of the first metal atoms to the second metal atoms is from 50:50 to 60:40.

9. The intermetallic compound according to claim 1, comprising a multi-coordinating atom that exhibits multi-coordination.

10. The intermetallic compound according to claim 9, wherein the multi-coordinating atom is at least one selected from the group consisting of nitrogen (N), oxygen (O), carbon (C), and boron (B).

11. The intermetallic compound according to claim 1, comprising at least one selected from the group consisting of an oxide of at least one of the first metal atom and the second metal atom, and a nitride of at least one of the first metal atom and the second metal atom.

12. A catalyst comprising

the intermetallic compound according to any one of claims 1 to 11, and
a support that carries the intermetallic compound.

13. A method for producing methanol, comprising bringing hydrogen and at least one of carbon monoxide and carbon dioxide into contact with the intermetallic compound according to any one of claims 1 to 11.

14. A method for producing carbon monoxide, comprising bringing hydrogen and carbon dioxide into contact with the intermetallic compound according to any one of claims 1 to 11.

15. A method for producing an intermetallic compound comprising a first metal atom and a second metal atom,

wherein the first metal atom is at least one selected from the group consisting of Pd, Rh, and Ir, and
the second metal atom is Mo,
the method comprising:

a first step of mixing a first compound containing the first metal atom and a second compound containing the second metal atom, dehydrating the mixture to obtain a dehydrated-product, and then carbonizing the dehydrated-product to obtain a carbide;
a second step of calcining the carbide obtained in the first step under air to obtain a calcined-product; and
a third step of subjecting the calcined-product obtained in the second step to a nitridation treatment in an $NH_3$ atmosphere.

16. The method for producing an intermetallic compound according to claim 15, wherein in the first step, the first compound and the second compound are mixed in the presence of citric acid.

17. The method for producing an intermetallic compound according to claim 15, wherein in the first step, the first compound and the second compound are mixed in the presence of polyvinylpyrrolidone.

FIG. 1

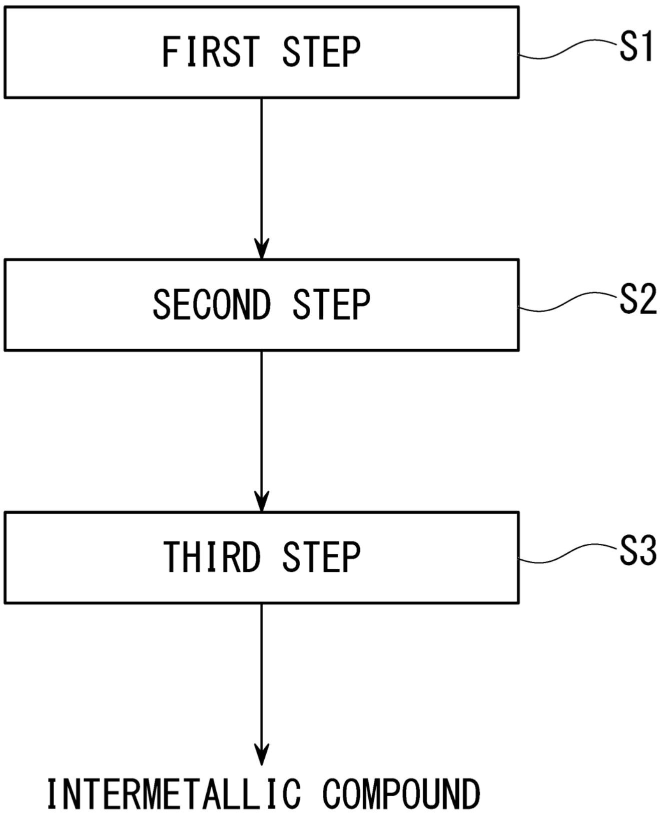
FIRST STEP
S1
SECOND STEP
S2
THIRD STEP
S3
INTERMETALLIC COMPOUND

FIG. 2

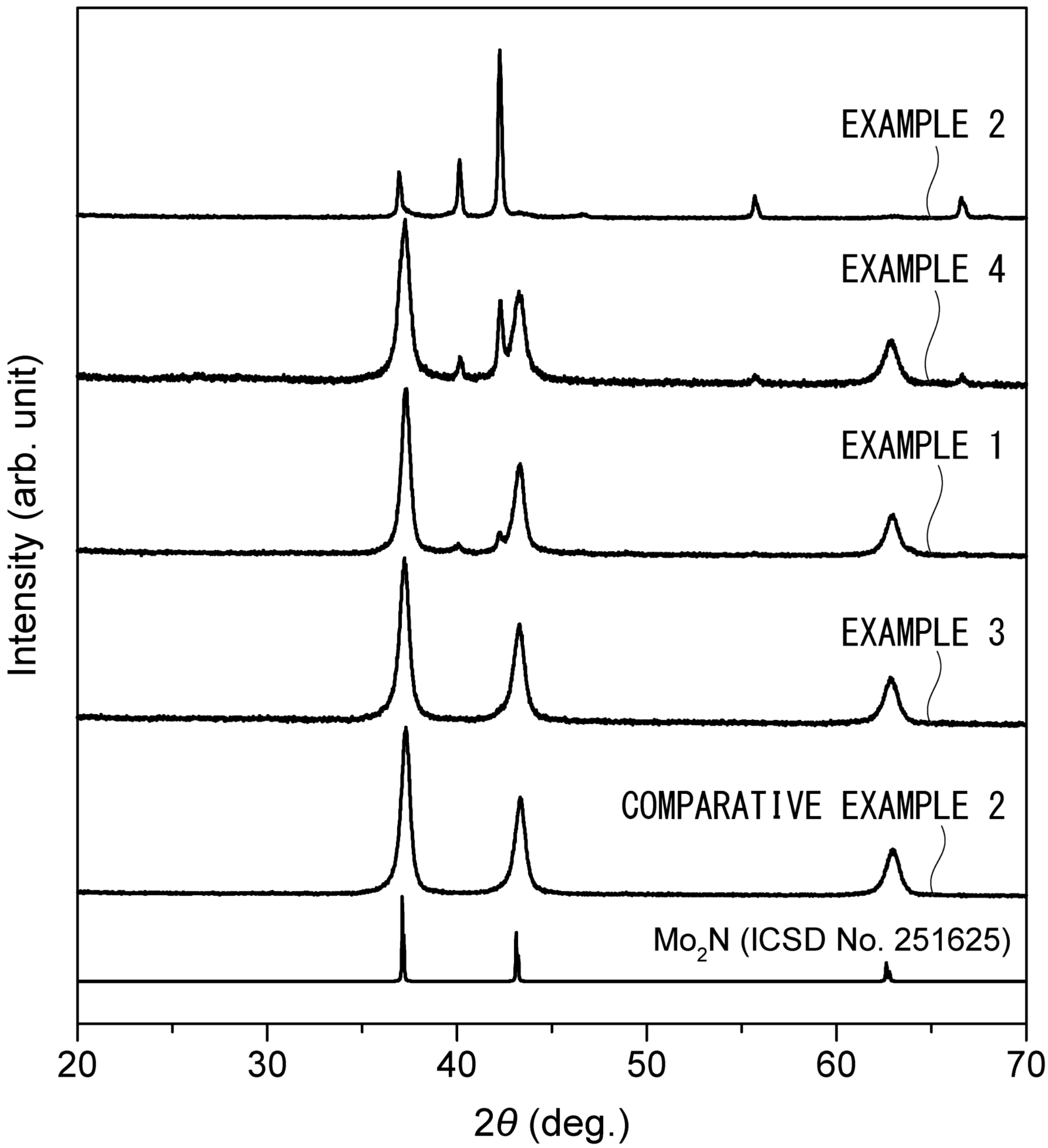
EXAMPLE 2
EXAMPLE 4
EXAMPLE 1
EXAMPLE 3
COMPARATIVE EXAMPLE 2
Mo2N (ICSD No. 251625)
Intensity (arb. unit)
20
30
40
50
60
70
2θ (deg.)

FIG. 3

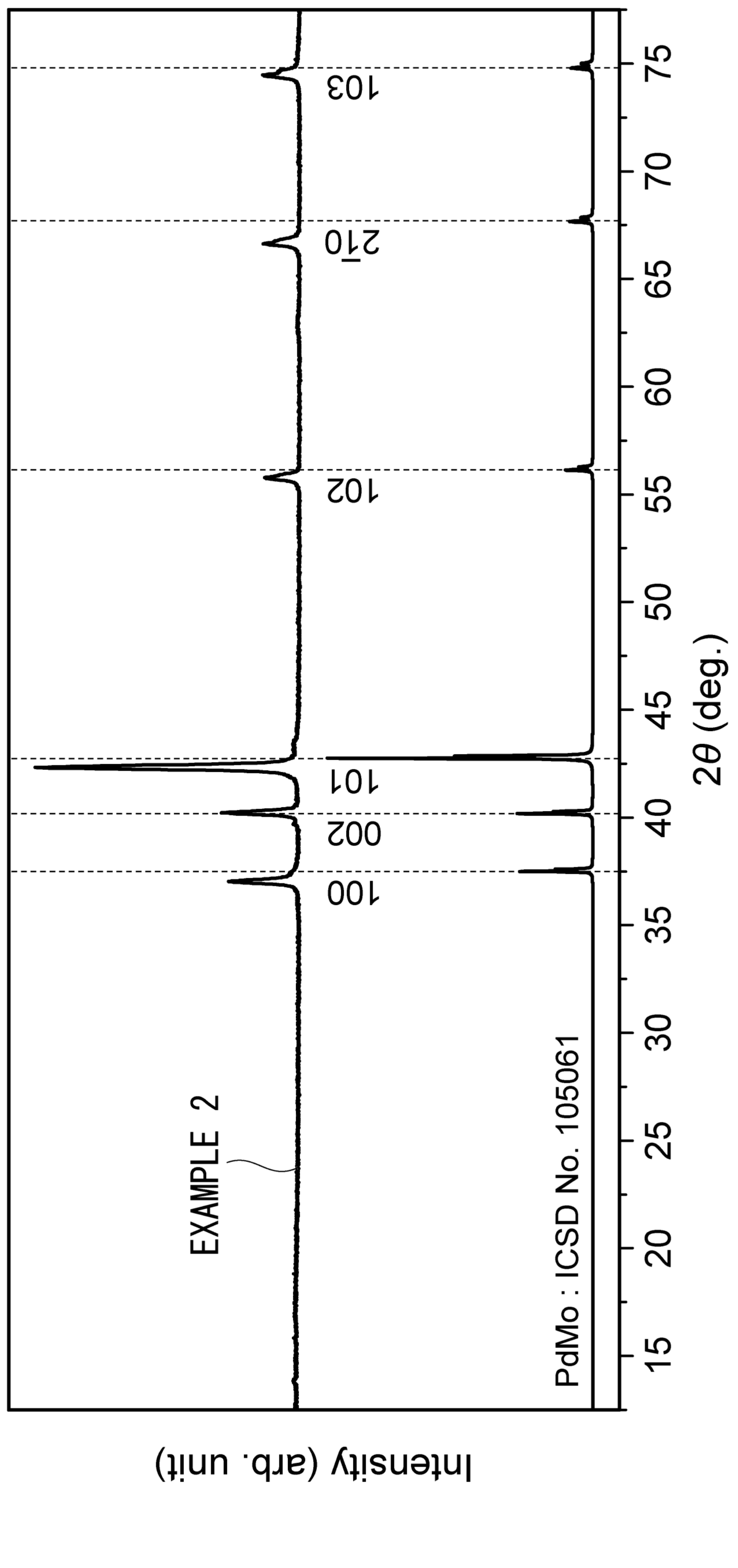
EXAMPLE 2
PdMo : ICSD No. 105061
100
002
101
102
2$\bar{1}$0
103
15
20
25
30
35
40
45
50
55
60
65
70
75
2θ (deg.)
Intensity (arb. unit)

FIG. 4

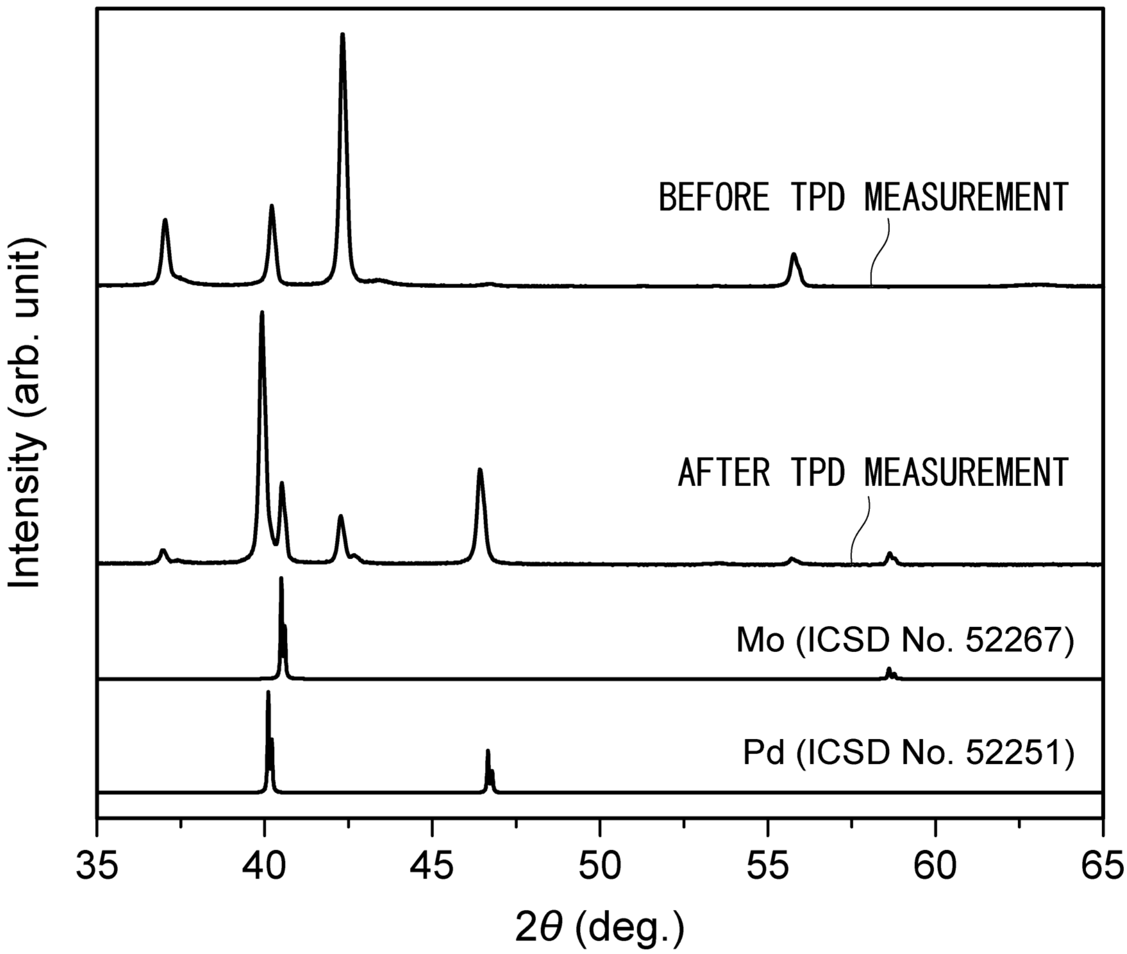
BEFORE TPD MEASUREMENT
AFTER TPD MEASUREMENT
Mo (ICSD No. 52267)
Pd (ICSD No. 52251)
Intensity (arb. unit)
35
40
45
50
55
60
65
2θ (deg.)

FIG. 5

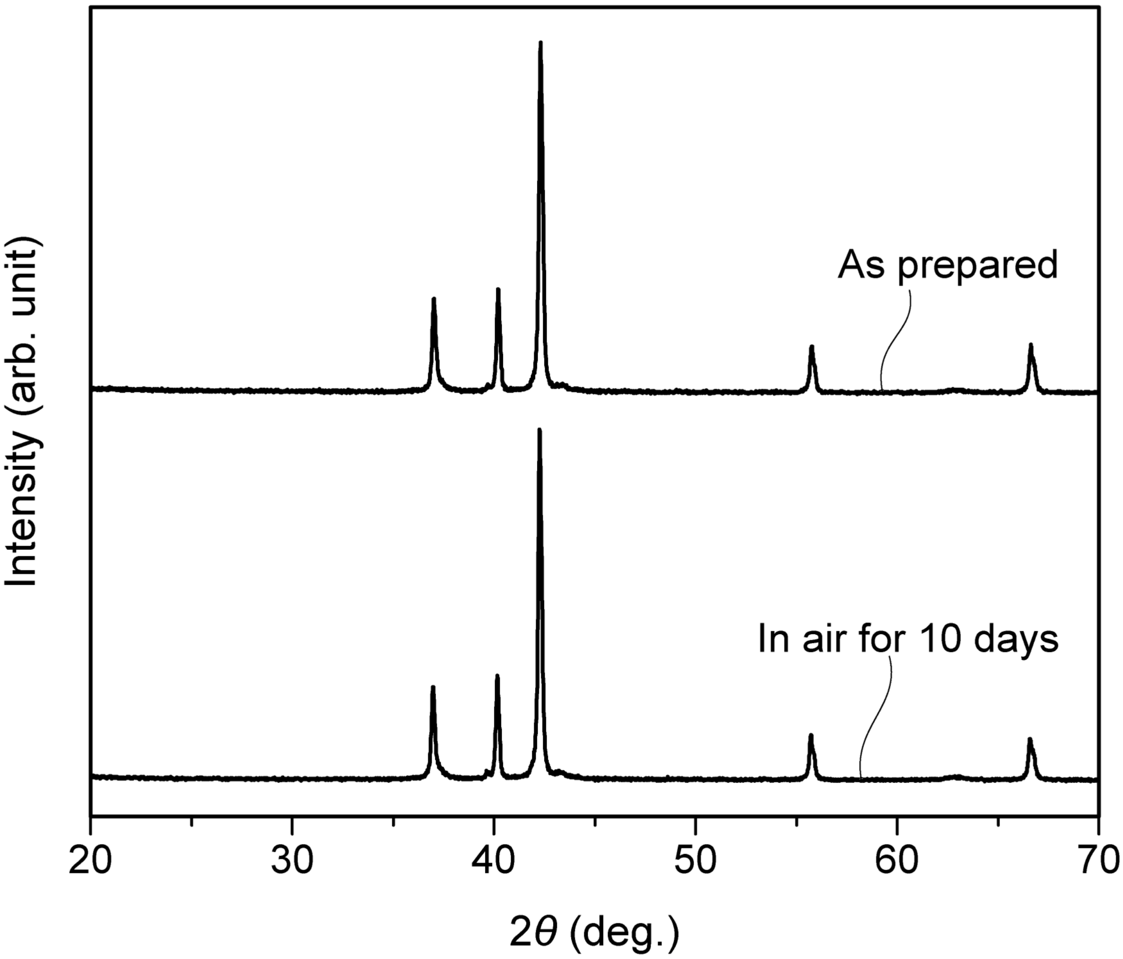
Intensity (arb. unit)
As prepared
In air for 10 days
20
30
40
50
60
70
2θ (deg.)

FIG. 6

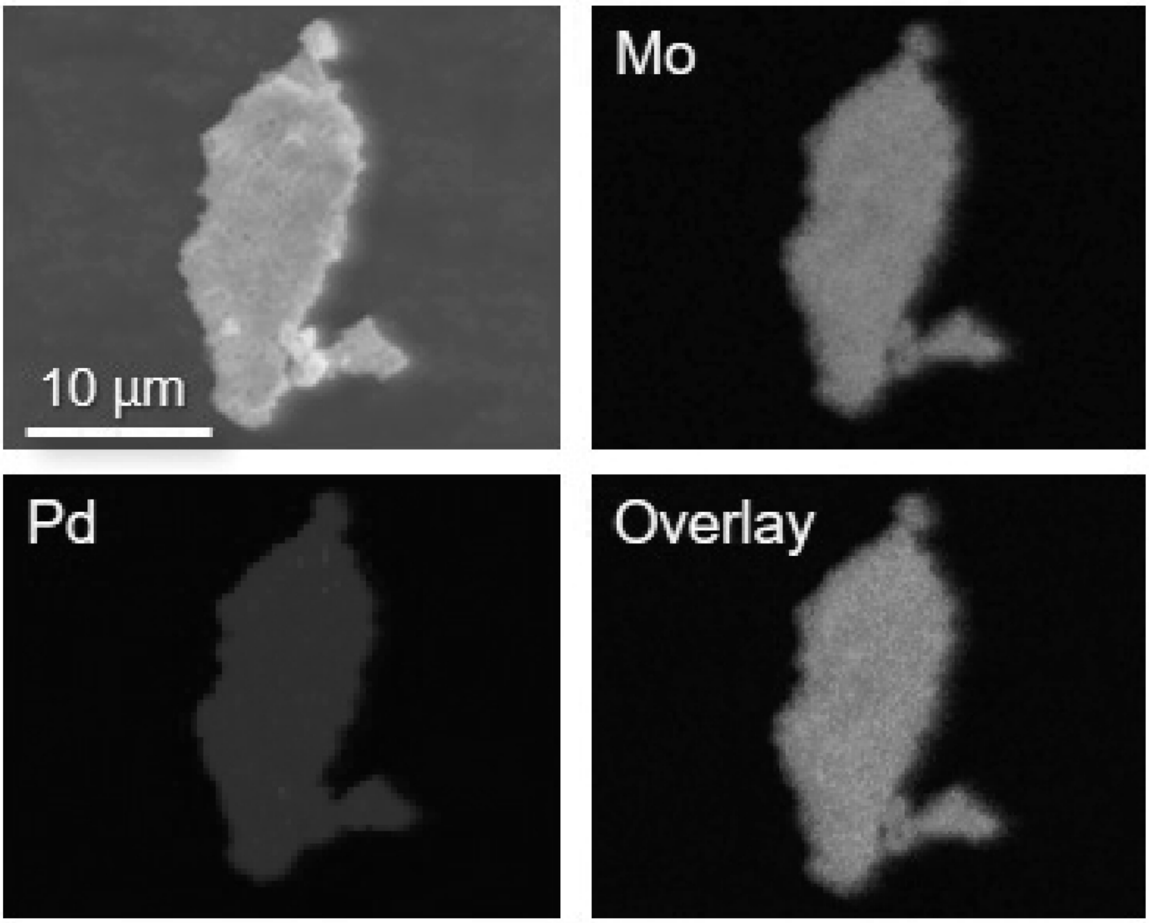

FIG. 7

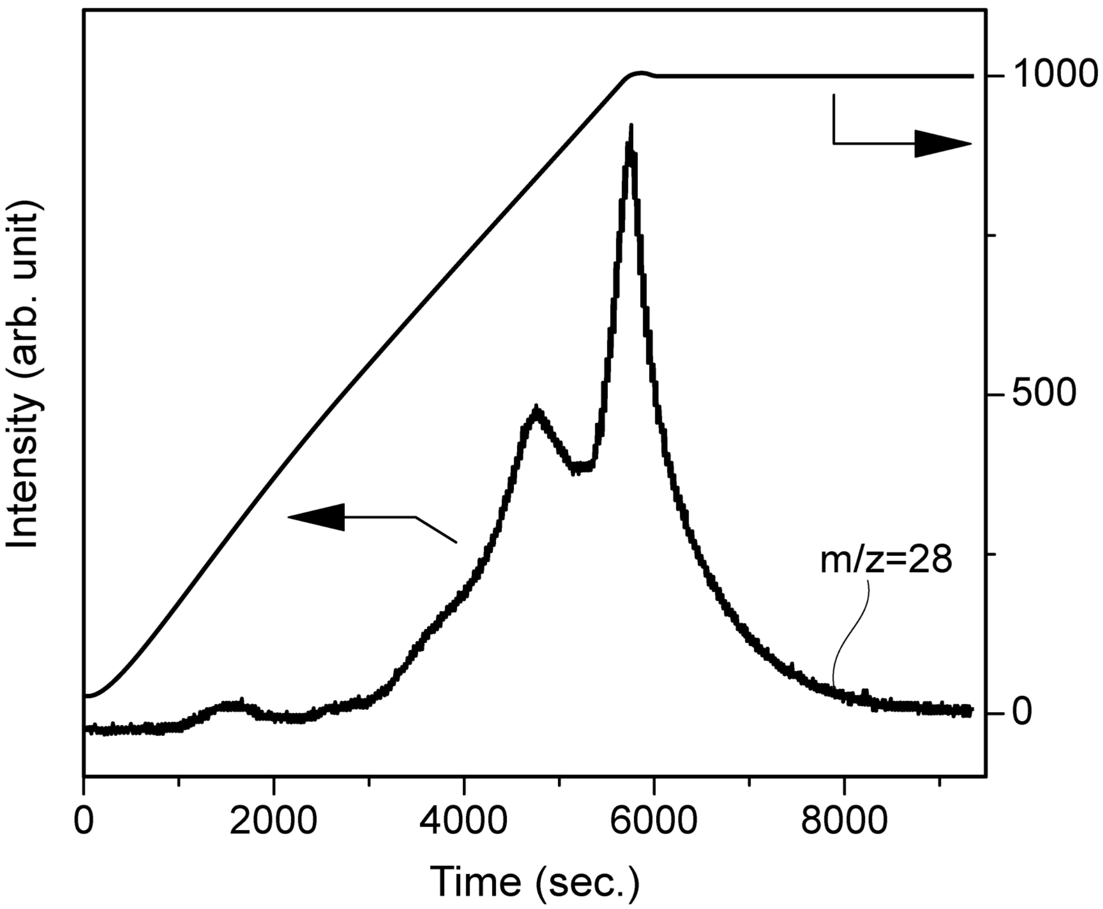
1000
500
0
Intensity (arb. unit)
Temperature (°C)
m/z=28
0
2000
4000
6000
8000
Time (sec.)

FIG. 8

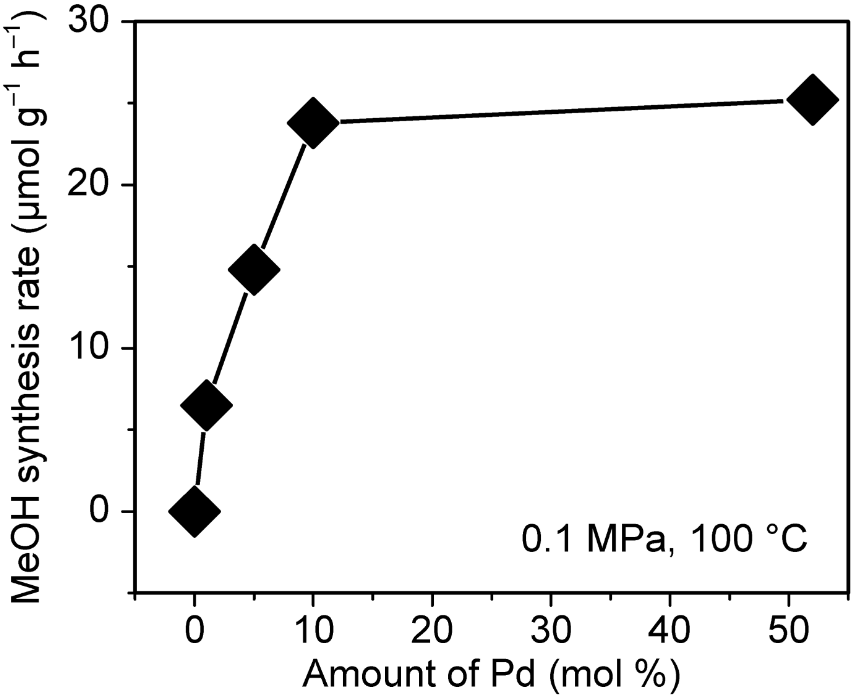
MeOH synthesis rate (μmol g⁻¹ h⁻¹)
30
20
10
0
0.1 MPa, 100 °C
0
10
20
30
40
50
Amount of Pd (mol %)

FIG. 9

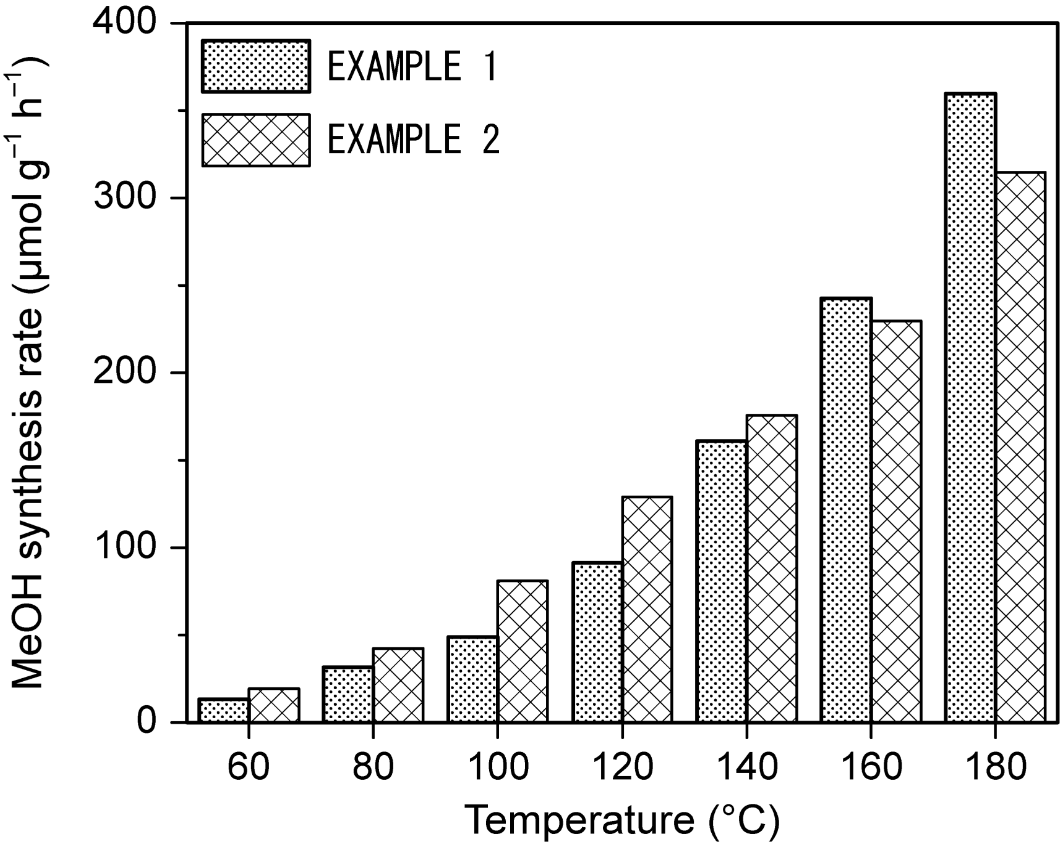
MeOH synthesis rate (μmol g⁻¹ h⁻¹)
400
300
200
100
0
EXAMPLE 1
EXAMPLE 2
60
80
100
120
140
160
180
Temperature (°C)

FIG. 10

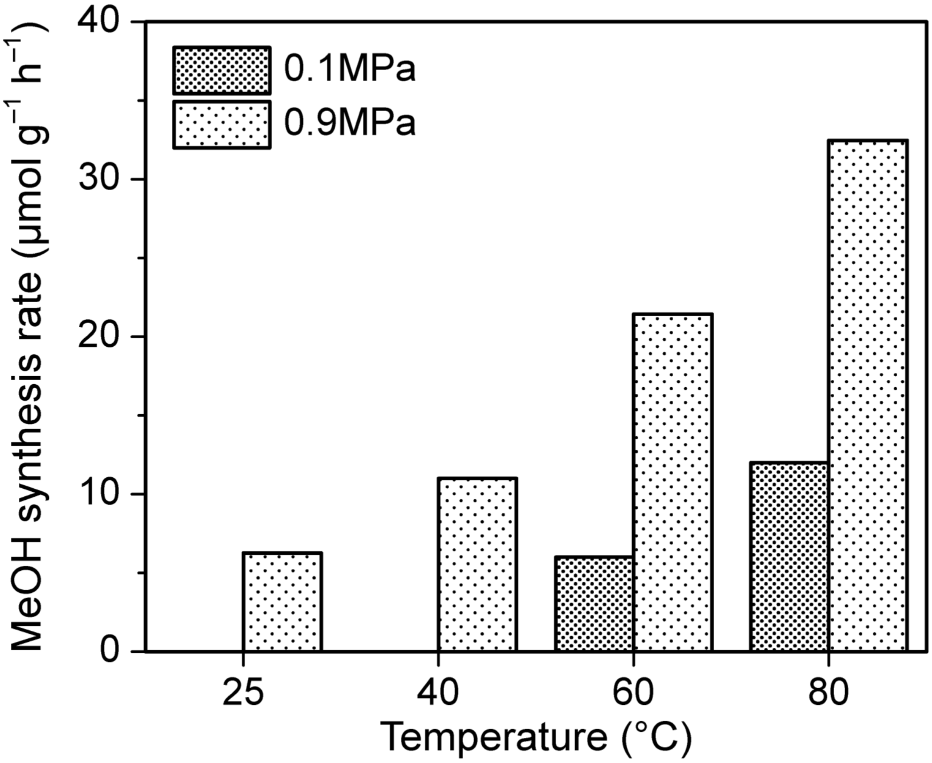
MeOH synthesis rate (μmol g⁻¹ h⁻¹)
0
10
20
30
40
0.1MPa
0.9MPa
25
40
60
80
Temperature (°C)

FIG. 11

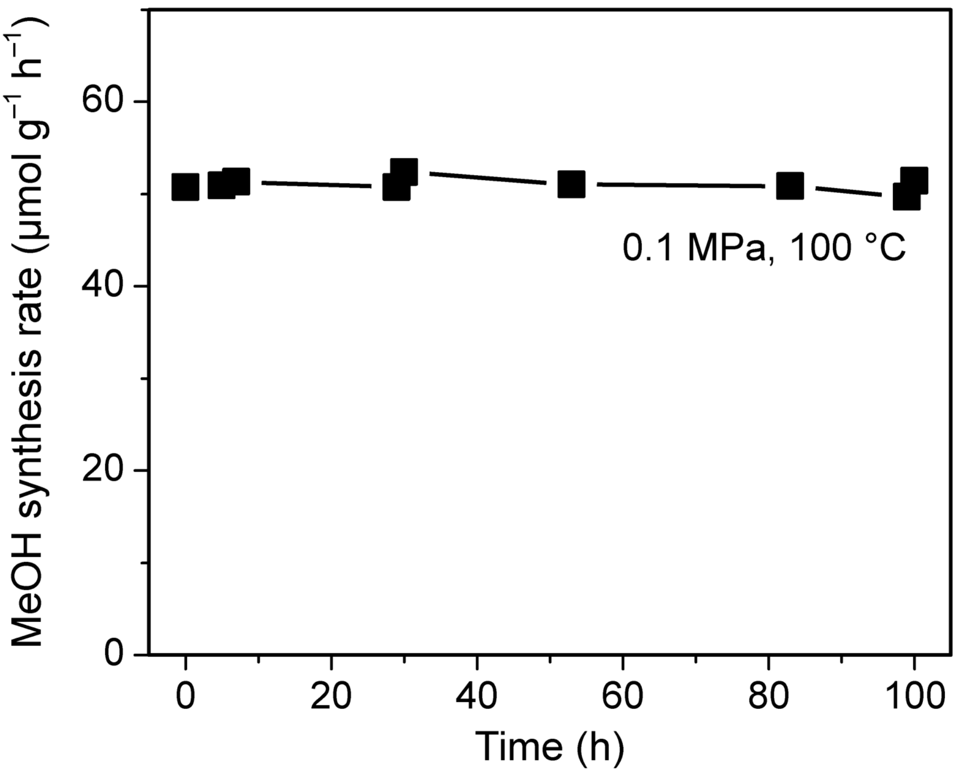
MeOH synthesis rate (μmol g⁻¹ h⁻¹)
0
20
40
60
0.1 MPa, 100 °C
0
20
40
60
80
100
Time (h)

FIG. 12

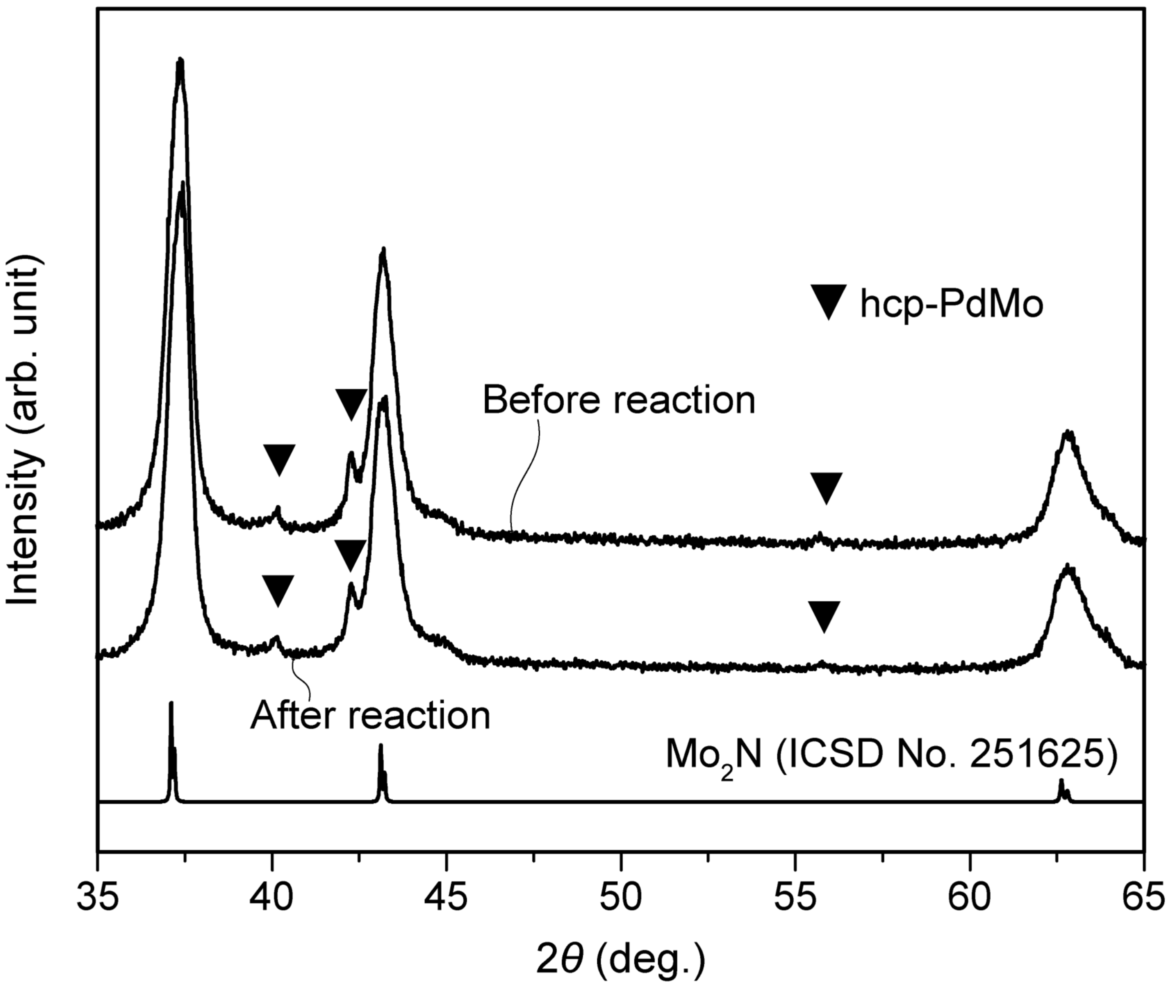
hcp-PdMo
Before reaction
After reaction
Mo2N (ICSD No. 251625)
Intensity (arb. unit)
35
40
45
50
55
60
65
2θ (deg.)

FIG. 13

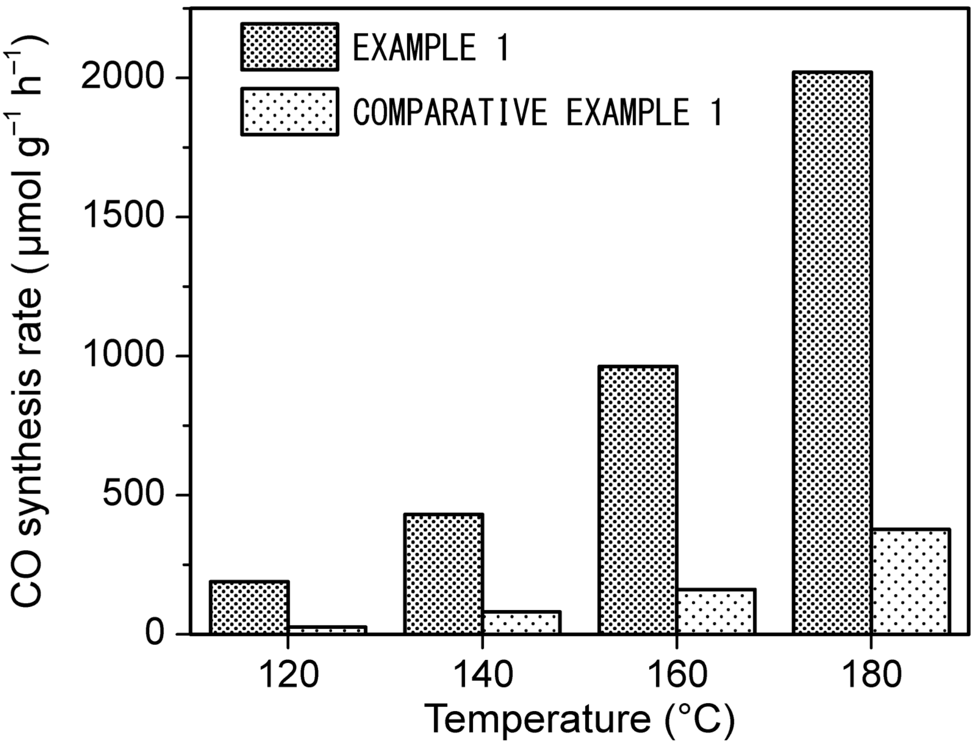
EXAMPLE 1
COMPARATIVE EXAMPLE 1
CO synthesis rate (μmol g$^{-1}$ h$^{-1}$)
0
500
1000
1500
2000
120
140
160
180
Temperature (°C)

FIG. 14

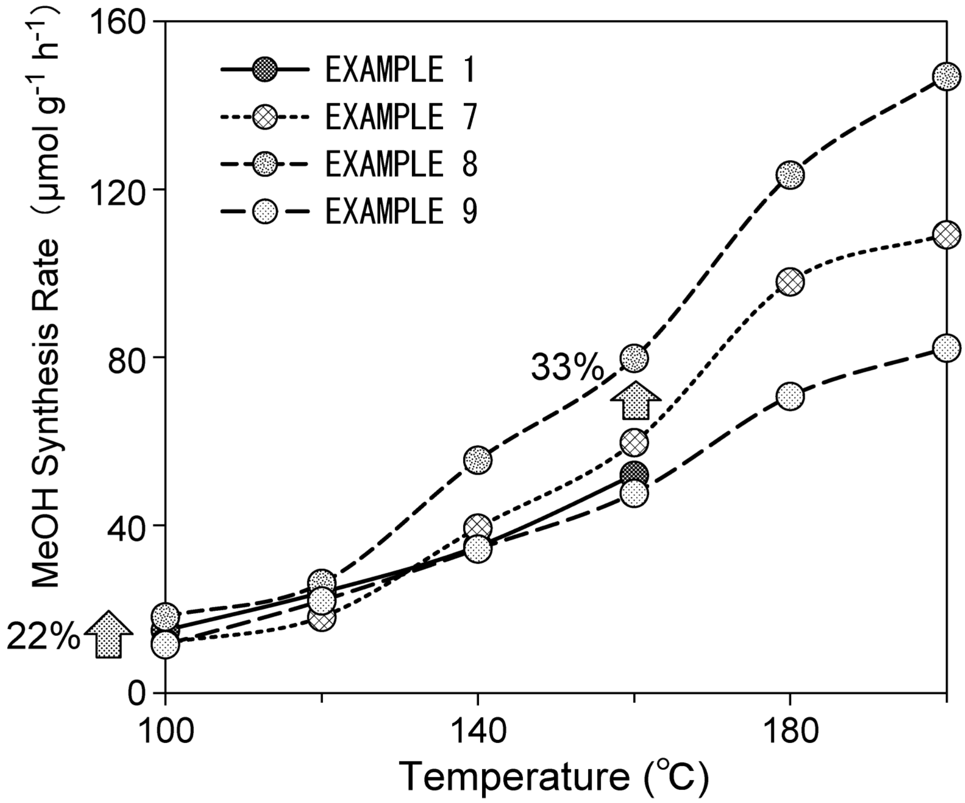
EXAMPLE 1
EXAMPLE 7
EXAMPLE 8
EXAMPLE 9
MeOH Synthesis Rate（μmol g$^{-1}$ h$^{-1}$）
0
40
80
120
160
33%
22%
100
140
180
Temperature (℃)

FIG. 15

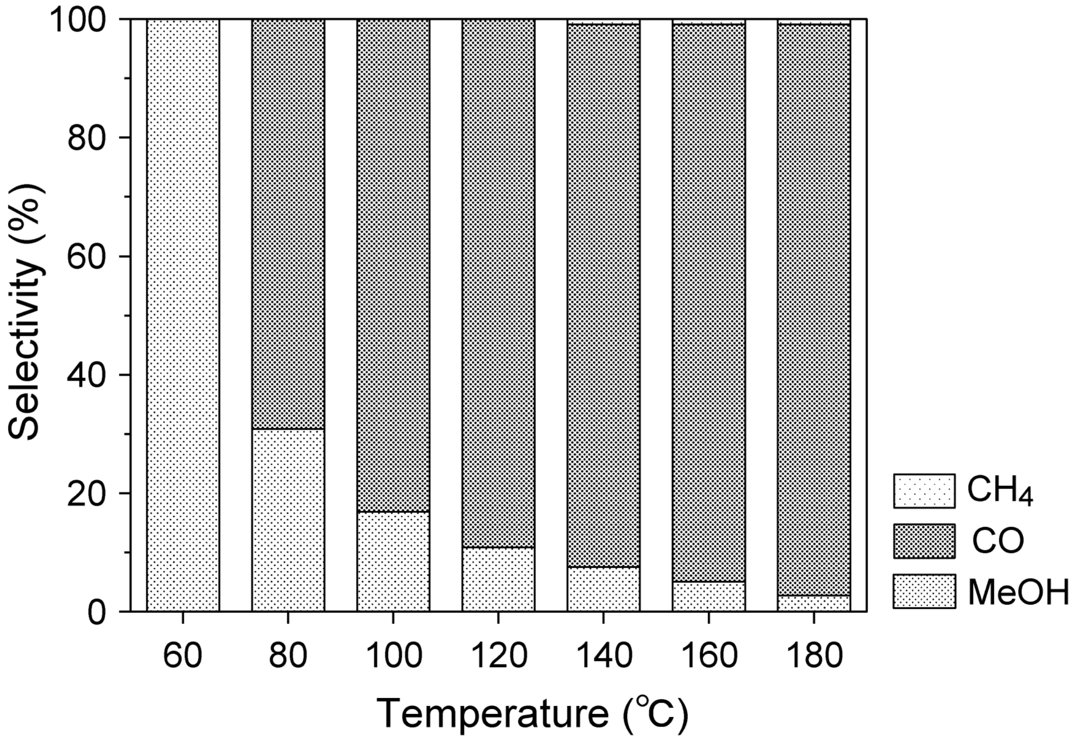
Selectivity (%)
0
20
40
60
80
100
60
80
100
120
140
160
180
Temperature (°C)
$CH_4$
CO
MeOH

FIG. 16

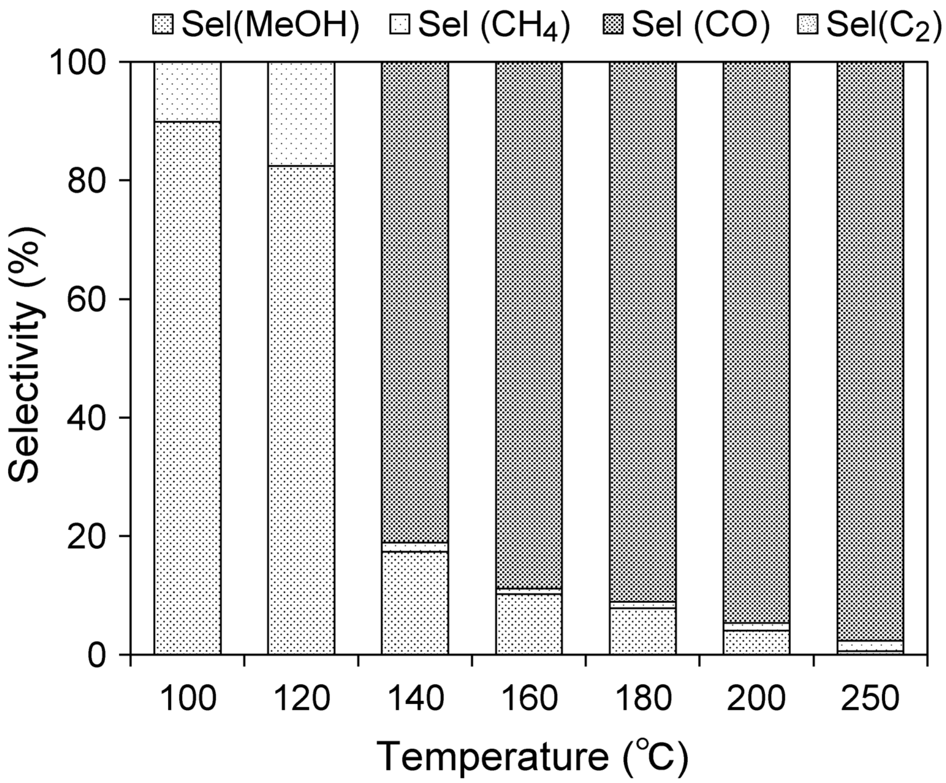
Sel(MeOH)
Sel ($CH_4$)
Sel (CO)
Sel($C_2$)
Selectivity (%)
0
20
40
60
80
100
100
120
140
160
180
200
250
Temperature (°C)

**INTERNATIONAL SEARCH REPORT**

International application No.
**PCT/JP2023/046981**

**A. CLASSIFICATION OF SUBJECT MATTER**

***B01J 27/24***(2006.01)i; ***B01J 37/04***(2006.01)i; ***B01J 37/08***(2006.01)i; ***B01J 37/16***(2006.01)i; ***C01B 32/40***(2017.01)i; ***C07B 61/00***(2006.01)i; ***C07C 29/157***(2006.01)i; ***C07C 31/04***(2006.01)i
FI: B01J27/24 M; B01J37/04 102; B01J37/08 ZAB; B01J37/16; C01B32/40; C07B61/00 300; C07C29/157; C07C31/04

According to International Patent Classification (IPC) or to both national classification and IPC

**B. FIELDS SEARCHED**

Minimum documentation searched (classification system followed by classification symbols)

B01J27/24; B01J37/04; B01J37/08; B01J37/16; C01B32/40; C07B61/00; C07C29/157; C07C31/04

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JST7580/JSTChina (JDreamIII)

**C. DOCUMENTS CONSIDERED TO BE RELEVANT**

| Category* | Citation of document, with indication, where appropriate, of the relevant passages | Relevant to claim No. |
|---|---|---|
| A | WO 2020/175309 A1 (OSAKA UNIVERSITY) 03 September 2020 (2020-09-03) entire text | 1-17 |
| A | JP 2008-013435 A (JFE ENGINEERING KK) 24 January 2008 (2008-01-24) entire text | 1-17 |
| A | JP 63-088043 A (TANAKA KIKINZOKU KOGYO KK) 19 April 1988 (1988-04-19) entire text | 1-17 |
| A | CN 114100661 A (FUZHOU UNIVERSITY) 01 March 2022 (2022-03-01) entire text | 1-17 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 February 2024** | **05 March 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2023/046981**

| Patent document cited in search report | Publication date (day/month/year) | Patent family member(s) | Publication date (day/month/year) |
|---|---|---|---|
| WO 2020/175309 A1 | 03 September 2020 | US 2022/0144789 A1<br>entire text<br>EP 3932545 A1 | |
| JP 2008-013435 A | 24 January 2008 | (Family: none) | |
| JP 63-088043 A | 19 April 1988 | (Family: none) | |
| CN 114100661 A | 01 March 2022 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2023007378 A


### Non-patent literature cited in the description


- **J. HU** ; **L. YU** ; **J. DENG** ; **Y. WANG** ; **K. CHENG** ; **C. MA** ; **Q. ZHANG** ; **W. WEN** ; **S. YU** ; **Y. PAN**. *Nat Catal*, 2021, vol. 4, 242-250